(19) Europäisches Patentamt
European Patent Office
Office européen des brevets

(11) Veröffentlichungsnummer: **0 300 966 B1**

# (12) EUROPÄISCHE PATENTSCHRIFT

(45) Veröffentlichungstag der Patentschrift: **14.10.92**

(51) Int. Cl.5: **C07C 259/04**, C07F 7/18, C07B 43/06, C07C 231/00, C07C 291/00

(21) Anmeldenummer: **88810480.9**

(22) Anmeldetag: **13.07.88**

## (54) Verfahren zur selektiven N-Acylierung von Amino-hydroxamsäurederivaten und darin verwendete Ausgangsstoffe.

(30) Priorität: **23.07.87 CH 2792/87**

(43) Veröffentlichungstag der Anmeldung:
**25.01.89 Patentblatt 89/04**

(45) Bekanntmachung des Hinweises auf die Patenterteilung:
**14.10.92 Patentblatt 92/42**

(84) Benannte Vertragsstaaten:
**AT BE CH DE ES FR GB GR IT LI LU NL SE**

(56) Entgegenhaltungen:
**WO-A-86/03745**
**DE-A- 1 163 337**
**DE-A- 1 186 076**

**CHEMIKER ZEITUNG 107. Jahrgang, Nr. 2, Februar 1983, WOLFGANG HEUCHEL et al.: "Silylierte Carbohydroxamsäuren"**

**METHODEN DER ORGANISCHEN CHEMIE, Band XIII/5, vierte Auflage, 1980, Georg Thieme Verlag, Stuttgart-New York; HOUBEN-WEYL: "Organo-Silicium-Verbindungen"**

**JOURNAL OF SYNTHETIC ORGANIC CHEMISTRY, April 87, Seiten 418-420, Georg Thieme Verlag, Stuttgart-New York, LUJIA NAKONIECZNA et al.: "A New Synthesis of N-Hydroxyamides Using Trimethylsilyl Protection"**

(73) Patentinhaber: **CIBA-GEIGY AG**
**Klybeckstrasse 141**
**CH-4002 Basel(CH)**

(72) Erfinder: **Peter, Heinrich, Dr.**
**Bündtenweg 69**
**CH-4102 Binningen(CH)**
Erfinder: **Moerker, Theophile**
**Ergolzstrasse 72**
**CH-4414 Füllinsdorf(CH)**

**EP 0 300 966 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Desferrioxamin B, der Grundstoff des Acylierungsverfahrens der vorliegenden Erfindung, ist bereits längere Zeit bekannt (H. Bickel, H. Keberle und E. Vischer: Kelv.Chim.Acta 46, 1385-9 [1963]). Seine chemische Struktur entspricht der Formel

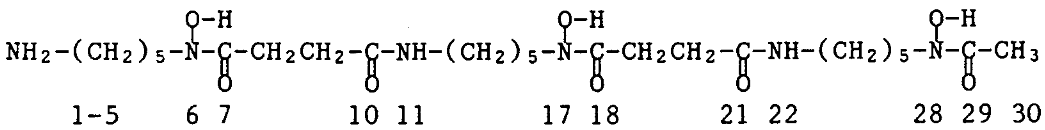

(A)

und wird im Einklang mit der Regel C-06 (Austausch-Nomenklatur) der offiziellen IUPAC-Nomenklatur mit dem systematischen Namen 6,17,28-Trihydroxy-7,10,18,21,29-pentaoxo-6,11,17,22,28-pentaazatriacont-ylamin bezeichnet. (Der Einfachheit halber werden jedoch nachfolgend die Namen der Derivate vom trivialen Namen abgeleitet, wobei die Lage einzelner Substituenten jeweils auf den Aminostickstoff N bzw. die als O, $O'$ und $O''$ bezeichneten Sauerstoffatome der Hydroxylgruppen in Stellungen 6, 17, bzw. 28 bezogen wird).

Zu den markantesten Eigenschaften von Desferrioxamin B und seinen Additionssalzen, die mit einem Aequivalent Säure gebildet werden, gehört die Fähigkeit, sich mit trivalenten Metallionen, wie Chrom(III)-, Aluminium- und in erster Linie Eisen(III)-Ionen, zu stabilen Metallkomplexen oder zu stabilen Chelat-artigen Addukten zu verbinden. Dies verleiht Desferrioxamin B die wertvolle pharmakologische Eigenschaft, die Ablagerung eisenhaltiger Pigmente im Gewebe zu verhindern und bei bestehenden Eisenablagerungen im Organismus eine Ausscheidung des Eisens zu bewirken, z.B. bei Hämochromatose, Hämosiderose, Lebercirrhose und Vergiftungen mit Verbindungen des dreiwertigen Eisens. Die breite therapeutische Anwendung von Desferrioxamin B und seinen Salzen (z.B. insbesondere vom Methansulfonat) erstreckt sich deshalb allgemein auf Krankheiten und krankhafte Zustände des menschlichen Körpers (sowie des Körpers anderer Warmblüter), welche mit einer übermässigen Belastung des Organismus mit Eisen(III)-Ionen ($Fe^{+++}$-Ionen) einhergehen, wie Thalassämie major, Sichelzell-Anämie, sideroachrestische Anämie, aplastische Anämie und weitere anämische Formen, in welchen Hämosiderose (d.h. eine lokale oder allgemeine Erhöhung der Eisenvorräte in sonst unbeschädigten Körpergeweben) eine Rolle spielt. Zu diesem Typus gehören auch krankhafte Zustände, die sich in Patienten nach mehrmaligen Bluttransfusionen oder mehrfach wiederholter Dialyse-Behandlung bei fehlender oder geschädigter Nierenfunktion entwickeln. Dank den komplexbildenden Eigenschaften erwies Desferrioxamin B eine bedeutende Wirksamkeit bei Erkrankungen durch Eisen(III)-abhängige Mikroorganismen und Parasiten, wie insbesondere bei Malaria, die nicht nur in der Humanmedizin, sondern auch in der Tiermedizin von prinzipieller Wichtigkeit ist. Auch die Komplex-Bildung mit anderen dreiwertigen Metallen kann zu deren Ausscheidung aus dem Organismus verwendet werden, z.B. zur Entfernung von Aluminium bei der Dialyse-Encephalopathie und Osteomalacia, sowie bei der Alzheimer Krankheit.

Bei einer solchen Vielfalt von Anwendungsmöglichkeiten ist es verständlich, dass man versucht, die günstigen physiologischen Eigenschaften von Desferrioxamin B als Grundsubstanz durch geeignetes Abwandeln für einzelne Einsatzgebiete spezifisch zu modifizieren und zu steigern. Dazu bietet sich insbesondere die chemische Abwandlung der zugänglichen funktionellen Gruppen, d.h. der endständigen Aminogruppe und/oder der Hydroxylgruppen der 3 Hydroxamsäure-Gruppierungen an, wobei es besonders wünschenswert wäre, zwischen den funktionellen Gruppen beider Typen unterscheiden zu können und nur eine Art von funktionellen Gruppen selektiv zu funktionalisieren. Die Acylierung stellt aus mehreren Gründen eine der begehrtesten Abwandlungen dar. Sie verläuft jedoch im Falle der weit überwiegenden Mehrzahl der einzuführenden Acylreste nicht selektiv. Im Gegensatz zu Aminocarbonsäuren können Aminohydroxamsäuren und insbesondere Aminooligohydroxamsäuren, wie Desferrioxamin B, nämlich in aller Regel nicht mit bekannten, direkten Acylierungsmethoden (z.B. nach Schotten-Baumann) selektiv an der Aminogruppe monoacyliert werden. Bei Verwendung äquimolarer oder leicht überschüssiger Mengen Acylierungsmittel werden meist sehr schwer trennbare Gemische erhalten, aus welchen das gewünschte N-Acylderivat nur in schlechter Ausbeute isoliert werden kann. Eine Ausnahme machen lediglich gewisse, einen Oxycarbonylrest, wie z.B. den tert.Butoxycarbonylrest, einführende Acylierungsmittel, wie Di-tert.butyl-dicarbonat, mit denen unter bestimmten Reaktionsbedingungen eine weitgehend selektive N-Acylierung von Desferrioxamin B gelingt. Zur Herstellung von N-Monoacylderivaten von Desferrioxamin B mit "normalen", d.h. von bestimmten Oxycarbonylresten verschiedenen Acylresten werden deshalb gemäss dem Stand der Technik

folgende Verfahren angewandt:

Durch Einsatz von überschüssigem Acylierungsmittel werden zunächst N,O,O′,O″-Tetraacylderivate hergestellt, aus welchen man durch verlustreiche selektive Ammonolyse und mühsames Auftrennen der erhaltenen Substanzgemische zu N-Monoacylaten gelangen kann. In wirtschaftlicher Hinsicht fällt je nach Art des Acylierungsmittels dabei ins Gewicht, dass nur 1/4 des eingesetzten Acylierungsmittels verwertet wird.

Falls man als Acylgruppe eine geeignete, acidolytisch oder hydrogenolytisch abspaltbare, veresterte Oxycarbonylgruppe (wie tert-Butoxycarbonyl bzw. Benzyloxycarbonyl) verwendet, so kann man ein solches N-Monoacylat mit einer anderen Säure an den freien Hydroxylgruppen acylieren und nach Abspalten der N-schützenden Acylgruppe zu O,O′,O″-Triacylaten mit freier Aminogruppe gelangen. Durch N-Acylierung der so erhaltenen O,O′,O″-Triacylate ist es möglich, zu N,O,O′,O″-Tetraacylaten mit verschiedenen Acylresten am Stickstoff einerseits und den Sauerstoffatomen andererseits zu gelangen. Die Herstellung von O-unsubstituierten N-Acylaten mit von Oxycarbonylresten verschiedenen beliebigen Acylresten ist jedoch nach dem letztgenannten Verfahren nicht möglich.

In einer weiteren Methode werden die Hydroxamsäurefunktionen von Desferrioxamin B durch vorgängige Komplexierung mit $Fe^{3+}$ oder $Al^{3+}$ gegen eine Acylierung geschützt. Durch diese Komplexierung wird jedoch auch die Reaktivität der Aminogruppe herabgesetzt. Daher verläuft die nachfolgende Acylierungsreaktion schleppend und mit unbefriedigender Ausbeute. In einer zusätzlichen Reaktionsstufe muss anschliessend das Metallion wieder entfernt werden.

Da die Herstellung eines N-Monoacylats nach den bisher bekannten Methoden umständlich ist und nur sehr unbefriedigende Resultate erbringt, besteht ein dringendes Bedürfnis nach einer einfachen und für alle Arten von Acylresten anwendbaren Methode, welche die selektive Einführung einer N-Acylgruppe in Desferrioxamin B auf einfache Weise und in hohen Ausbeuten ermöglicht und zu reinen Produkten führt.

Die erfindungsgemässe Lösung dieser Aufgabe beruht auf dem überraschenden Befund, dass ein neues, durch organische Silylgruppen am Aminostickstoff und an den Hydroxylsauerstoffen geschütztes Derivat von Desferrioxamin B überraschenderweise mit einer breiten Palette von konventionellen organischen Acylierungsmitteln unter üblichen Reaktionsbedingungen reagiert und sogar so, dass dadurch selektiv die N-silylierte Aminogruppe acyliert wird, wogegen die an den Sauerstoffatomen silylierten Hydroxygruppen unverändert bleiben. Da die benötigten neuen N- und O-silyierten Ausgangsstoffe einfach und praktisch quantitativ aus der unsubstituierten Grundverbindung erhältlich sind und weil die O-Silylgruppen leicht, z.B. durch milde Solvolyse, abspaltbar sind, weist das erfindungsgemässe Verfahren alle Vorteile auf, welche bei den früheren Lösungen gefehlt haben. Die erzielte Ausbeute und der Reinheitsgrad der gewünschten Verbindungen sind so hoch, dass es sich sogar als erheblich vorteilhafter erwiesen hat, ein N,O,O',O"-Tetracylderivat von Desferrioxamin B mit vier identischen Acylresten statt über die direkte Tetraacylierung von Desferrioxamin B auf dem indirekten Weg über das gemäss dem erfindungsgemässen Verfahren erhältliche Mono-N-Acyl-Derivat herzustellen.

Die vorliegende Erfindung betrifft ein Verfahren zur Einführung eines organischen Acylrestes selektiv an das Stickstoffatom der Aminogruppe von Desferrioxamin B oder eines teilweise O-acylierten Derivats davon, dadurch gekennzeichnet, dass man eine Verbindung der Formel II, worin

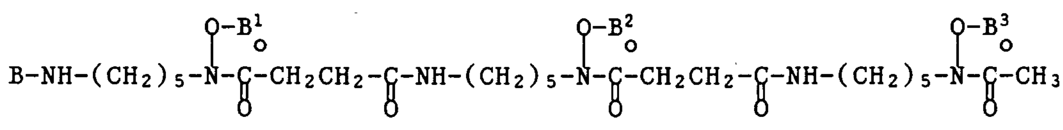

(II)

B eine organische Silylgruppe (Sil) der Formel

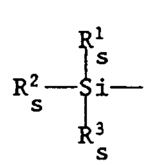

(Sil)

ist, in welcher $R_s^1$ und $R_s^2$ unabhängig voneinander je unsubstituiertes $C_{1-8}$-Hydrocarbyl und $R_s^3$ unsubstituiertes $C_{1-8}$-Hydrocarbyl oder Chlor bedeuten, und mindestens eines der Symbole $B_o^1$ , $B_o^2$ und $B_o^3$ für eine

organische Silylgruppe Sil steht und die übrigen, jedes unabhängig von anderen, für Sil oder für einen organischen Acylrest Ac stehen, mit einem einen Acylrest Ac einführenden Mittel umsetzt und die vorhandenen O-gebundenen Silylgruppen Sil solvolytisch abspaltet.

Die durch das erfindungsgemässe Verfahren erhältlichen Verbindungen sind insbesondere diejenigen der Formel

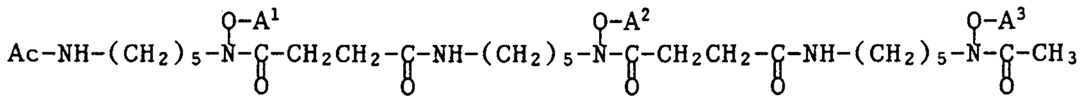

(I)

worin Ac die oben angegebene Bedeutung hat und mindestens eines der Symbole $A^1$, $A^2$ und $A^3$ für Wasserstoff steht und die übrigen, je unabhängig von den anderen, einen unten näher definierten Acylrest Ac bedeuten.

In bevorzugten Ausgangsstoffen der Formel II stehen B, $B_o^1$ , $B_o^2$ und $B_o^3$ alle für eine organische Silylgruppe Sil derselben Bedeutung. In diesem Fall resultiert als Produkt ein N-Monoacylat von Desferrioxamin B der Formel I, worin $A^1$, $A^2$ und $A^3$ je Wasserstoff bedeuten.

Die in der organischen Silylgruppe Sil vorhandenen Hydrocarbylreste $R_s^1$ und $R_s^2$ sind insbesondere $C_{1-8}$-Alkylreste, beispielsweise Hexyl, 4-Methylpentyl, Pentyl, Ethyl und vor allem Methyl, ferner auch Aryl- und Aralkylreste, beispielsweise Phenyl oder p-Tolyl, bzw. Benzyl oder Phenethyl; vorzugsweise sind beide Reste gleich. Das Symbol $R_s^3$ kann für Chlor stehen oder eine der genannten Bedeutungen der Symbole $R_s^1$ und $R_s^2$ haben, wobei alle 3 Symbole vorzugsweise dieselbe Bedeutung haben; und vor allem bedeutet $R_s^3$ Methyl.

Eine geeignete organische Silylgruppe Sil ist beispielsweise Tributylsilyl, Tribenzylsilyl, Phenyl-dimethylsilyl, Benzyl-dimethylsilyl, Hexyl-dimethylsilyl, tert-Butyl-dimethylsilyl, Triethylsilyl, Diethyl-chlorsilyl und insbesondere Dimethyl-chlorsilyl und vor allem Trimethylsilyl.

Der organische Acylrest Ac leitet sich von einer gegebenenfalls funktionell abgewandelten Carbonsäure, einer organischen Sulfonsäure oder einer veresterten Phosphorsäure ab und weist vorzugsweise höchstens 40 C-Atome auf.

Ein von einer Carbonsäure abgeleiteter Acylrest Ac ist insbesondere einer der Teilformel Z-X-C(=O)- ($Ac^1$), worin X eine einfache Bindung, Oxy oder gegebenenfalls substituiertes Imino und Z Hydrocarbyl $R^o$ oder, falls X eine einfache Bindung ist, auch Wasserstoff, Chlor oder 1-Imidazolyl darstellt. Entsprechend der Bedeutung von X leiten sich die Acylreste von Hydrocarbylcarbonsäuren, Monoestern bzw. Monoamiden der Kohlensäure ab, von Ameisensäure (Formyl), Chlorameisensäure (Chloroformyl) bzw. 1-Imidazolylcarbonsäure.

Acylreste von Hydrocarbylcarbonsäuren sind insbesondere durch die Teilformel $R_b^o$ -C(=O)- ($Ac_a^1$ ) charakterisiert, worin $R_b^o$ entweder für Wasserstoff steht (und somit den Formylrest bildet) oder Hydrocarbyl $R^o$ darstellt (und somit den Rest einer gegebenenfalls substituierten acyclischen, carbocyclischen, carbocyclisch-acyclischen, heterocyclischen oder heterocyclisch-acyclischen Monocarbonsäure bildet.

Hydrocarbyl $R^o$ ist ein acyclischer (aliphatischer), carbocyclischer oder carbocyclisch-acyclischer Kohlenwasserstoffrest, der insgesamt bis zu 60, vorzugsweise höchstens 40, insbesondere höchstens 20, Kohlenstoffatome hat und gesättigt oder ungesättigt, unsubstituiert oder substituiert sein kann. Er kann auch anstelle von einem, zwei oder mehreren Kohlenstoffatomen gleiche oder verschiedene Heteroatome, wie insbesondere Sauerstoff, Schwefel und Stickstoff, im acyclischen und/oder cyclischen Teil enthalten; im letzteren Fall wird er als ein heterocyclischer Rest (Heterocyclylrest) oder ein heterocyclisch-acyclischer Rest bezeichnet.

Ungesättigte Reste sind solche, die eine oder mehrere Mehrfachbindungen (Doppel- und/oder Dreifachbindungen) enthalten. Cyclische Reste, worin mindestens ein 6gliedriger carbocyclischer oder ein 5- bis 8gliedriger heterocyclischer Ring die maximale Anzahl nichtcumulierter Doppelbindungen enthält, werden als aromatisch bezeichnet. Carbocyclische Reste, worin mindestens ein Ring als ein 6gliedriger aromatischer Ring (d.h. Benzolring) vorliegt, werden als Arylreste bezeichnet.

Wenn nicht anders angegeben, enthalten in der vorliegenden Offenbarung mit dem Präfix "Nieder" bezeichnete organische Reste höchstens 7, vorzugsweise höchstens 4 Kohlenstoffatome.

Ein acyclischer Kohlenwasserstoffrest ist insbesondere ein gerader oder verzweigter Niederalkyl-, Niederalkenyl-, Niederalkadienyl-oder Niederalkynylrest. Niederalkyl ist z.B. Methyl, Ethyl, n-Propyl, Isopropyl, n-Butyl, Isobutyl, sek-Butyl oder tert-Butyl, weiter auch n-Pentyl, Isopentyl, n-Hexyl, Isohexyl und n-Heptyl; Niederalkenyl ist z.B. Allyl, Propenyl, Isopropenyl, 2- oder 3-Methallyl und 2-oder 3-Butenyl; Niederalkynyl ist z.B. Propargyl oder 2-Butynyl. Ein acyclischer Kohlenwasserstoffrest ist inbesondere auch ein gerader Alkyl- oder Alkenylrest mit 10-20 C-Atomen.

Ein carbocyclischer Kohlenwasserstoffrest ist insbesondere ein mono-, bi-oder polycyclischer Cycloalkyl-, Cycloalkenyl- oder Cycloalkadienylrest, oder ein entsprechender, aromatische Ringe enthaltender Arylrest. Bevorzugt sind Reste mit höchstens 12 Ringkohlenstoffatomen und 3- bis 8-, vorzugsweise 5- bis 7-, vor allem 6gliedrigen Ringen, wobei sie auch einen oder mehrere acyclische Reste, z.B. die oben genannten, und insbesondere die Niederalkylreste, oder weitere carbocyclische Reste, tragen können. Carbocyclisch-acyclische Reste sind solche, in welchen ein acyclischer Rest, insbesondere einer mit höchstens 7, vorzugsweise höchstens 4 Kohlenstoffatomen, wie vor allem Methyl, Ethyl und Vinyl, einen oder mehrere carbocyclische, gegebenenfalls aromatische Reste der obigen Definition trägt. Insbesondere sind Cycloalkyl-niederalkyl-und Arylniederalkylreste, sowie ihre im Ring und/oder Kette ungesättigten Analogen, welche den Ring am endständigen C-Atom der Kette tragen, zu erwähnen.

Cycloalkyl ist z.B. Cyclopropyl, Cyclobutyl, Cyclopentyl, Cyclohexyl, Cycloheptyl und Cyclooctyl, sowie Bicyclo[2,2,2]octyl, 2-Bicyclo[2,2,1]heptyl und Adamantyl, welche auch durch 1, 2 oder mehrere Alkylreste, vor allem Methylreste, substituiert sein können; Cycloalkenyl ist z.B. einer der bereits genannten monocyclischen Cycloalkylreste, der eine Doppelbindung in 1-, 2- oder 3-Stellung trägt. Cycloalkyl-niederalkyl oder -niederalkenyl ist z.B. ein durch einen der oben genannten Cycloalkylreste substituierter Methyl-, 1- oder 2-Ethyl-, 1- oder 2-Vinyl, 1-, 2- oder 3-Propyl- bzw. Allylrest, wobei die am Ende der linearen Kette substituierten Reste bevorzugt sind.

Ein Arylrest ist in erster Linie ein Phenyl, ferner ein Naphthyl-, wie 1- oder 2-Naphthyl-, ein Biphenylyl-, wie insbesondere 4-Biphenylyl-, weiter auch ein Anthryl-, Fluorenyl- oder Azulenylrest, sowie ihre Analogen mit einem oder mehreren gesättigten Ringen. Bevorzugte Arylniederalkyl- oder -niederalkenyl-Reste sind z.B. Phenylniederalkyl oder Phenyl-niederalkenyl mit endständigem Phenylrest, wie z.B. Benzyl, Phenethyl, 1-, 2- oder 3-Phenylpropyl, Diphenylmethyl (Benzhydryl), Trityl bzw. Styryl und Cinnamyl, ferner auch 1- oder 2-Naphthylmethyl.

Unter Arylresten, die acyclische Reste tragen, sind insbesondere o-, m- und p-Tolyl und Xylylreste mit verschieden situierten Methylresten zu erwähnen.

Heterocyclische Reste, einschliesslich heterocyclisch-acyclischer Reste, sind insbesondere monocyclische, aber auch bi- oder polycyclische, aza-, thia-, oxa-, thiaza-, oxaza-, diaza-, triaza- oder tetraza-Reste aromatischen Charakters, sowie entsprechende partiell oder in erster Linie ganz gesättigte heterocyclische Reste dieser Art, wobei solche Reste gegebenenfalls, z.B. wie die obgenannten carbocyclischen oder Arylreste, weitere acyclische, carbocyclische oder heterocyclische Reste tragen können und/oder durch funktionelle Gruppen mono-, di- oder polysubstituiert sein können. Der acyclische Teil in heterocyclisch-acyclischen Resten hat z.B. die für die entsprechenden carbocyclisch-acyclischen Reste gegebene Bedeutung. Sofern sich ein Heterocyclylrest als direkter Substituent $R^o$ an einem Heteroatom, wie Sauerstoff oder Stickstoff, befindet, so muss seine freie Valenz von einem seiner C-Atome ausgehen. In erster Linie sind es unsubstituierte oder substituierte monocyclische Reste mit einem Stickstoff-, Sauerstoff- oder Schwefelatom, wie 2-Aziridinyl, und insbesondere aromatische Reste dieser Art, wie Pyrryl, z.B. 2-Pyrryl oder 3-Pyrryl, Pyridyl, z.B. 2-, 3- oder 4-Pyridyl, ferner Thienyl, z.B. 2- oder 3-Thienyl, oder Furyl, z.B. 2-Furyl; analoge bicyclische Reste mit einem Stickstoff-, Sauerstoff- oder Schwefelatom sind z.B. Indolyl, wie 2- oder 3-Indolyl, Chinolyl, wie 2- oder 4-Chinolyl, Isochinolyl, Bezofuranyl, Chromenyl oder Benzothienyl; bevorzugte monocyclische und bicyclische Reste mit mehreren Heteroatomen sind z.B. Imidazolyl, wie 2-Imidazolyl, Pyrimidinyl, wie 2- oder 4-Pyrimidinyl, Oxazolyl, Isoxazolyl oder Thiazolyl bzw. Benzimidazolyl, Benzoxazolyl oder Chinazolyl. Entsprechende partiell oder, insbesondere, ganz gesättigte analoge Reste kommen auch in Betracht, wie 2-Tetrahydrofuryl, 2- oder 3-Pyrrolidyl, 2-, 3-, oder 4-Piperidyl, sowie auch 2- oder 3-Morpholinyl, 2- oder 3-Thiomorpholinyl, 2-Piperazinyl und N,N′-Bisniederalkyl-2-piperazinyl-Reste. Diese Reste können auch einen oder mehrere acyclische, carbocyclische oder heterocyclische Reste, insbesondere die obengenannten, tragen. Heterocyclisch-acyclische Reste sind insbesondere von acyclischen Resten mit höchstens 7, vorzugsweise höchstens 4 Kohlenstoffatomen, z.B. von den obengenannten, abgeleitet und können einen, zwei oder mehrere heterocyclische Reste, z.B. die obengenannten, tragen, wobei der Ring zur Kette auch durch einen seiner Stickstoffatome gebunden werden kann.

Wie bereits erwähnt wurde, kann ein Hydrocarbyl (einschliesslich eines Heterocyclyls) $R^o$ durch einen, zwei oder mehrere gleichartige oder verschiedenartige Substituenten (funktionelle Gruppen) substituiert sein; die folgenden Substituenten kommen insbesondere in Betracht: freie, veretherte und veresterte Hydroxylgruppen; Mercapto- sowie Niederalkylthio- und gegebenenfalls substituierte Phenylthiogruppen; Halogenatome, wie Chlor und Fluor, aber auch Brom und Iod; Oxogruppen, welche in der Form von Formyl- (d.h. Aldehydo-) und Keto-gruppen, auch als entsprechende Acetale bzw. Ketale vorliegen; Azido- und

Nitrogruppen; primäre, sekundäre und vorzugsweise tertiäre Aminogruppen, durch konventionelle Schutzgruppen geschützte primäre oder sekundäre Aminogruppen, Acylaminogruppen und Diacylaminogruppen, sowie gegebenenfalls funktionell abgewandelte Sulfogruppen, wie Sulfamoyl- oder in Salzform vorliegende Sulfogruppen. Alle diese funktionellen Gruppen dürfen sich nicht am C-Atom befinden, von dem die freie Valenz zu einem Heteroatom, wie insbesondere Sauerstoff, Schwefel oder Stickstoff, ausgeht, vorzugsweise sind sie von dieser freien Valenz (und somit vom Heteroatom) durch 2 oder auch mehrere C-Atome getrennt. Der Hydrocarbylrest kann auch freie und funktionell abgewandelte Carboxylgruppen, wie in Salzform vorliegende oder veresterte Carboxylgruppen, gegebenenfalls einen oder zwei Kohlenwasserstoffreste tragende Carbamoyl-, Ureidocarbonyl- oder Guanidinocarbonylgruppen, und Cyangruppen tragen.

Eine als Substituent im Hydrocarbyl vorliegende veretherte Hydroxylgruppe ist z.B. eine Niederalkoxygruppe, wie die Methoxy-, Ethoxy-, Propoxy-, Isopropoxy-, Butoxy- und tert-Butoxygruppe, welche auch substituierte sein kann. So kann eine solche Niederalkoxygruppe durch Halogenatome, insbesondere in 2-Stellung, wie im 2,2,2-Trichlorethoxy-, 2-Chlorethoxy- oder 2-Iodethoxyrest, oder durch Niederalkoxyreste, insbesondere in 2-Stellung, wie im 2-Methoxyethoxyrest, substituiert sein. Eine besonders bevorzugte Ausgestaltung der veretherten Hydroxylgruppen liegt in Oxaalkylresten vor, in welchen ein, vorzugsweise lineares, Alkyl anstelle mehrerer C-Atome Sauerstoffatome enthält, die vorzugsweise durch mehrere (vor allem 2) C-Atome voneinander getrennt sind, so dass sie eine, gegebenenfalls mehrfach sich wiederholende Gruppe $(-O-CH_2CH_2)_{\overline{n}}$, worin n = 1 bis 14 ist, bilden. Ferner sind solche veretherten Hydroxylgruppen auch gegebenenfalls substituierte Phenoxyreste und Phenylniederalkoxyreste, wie vor allem Benzyloxy, Benzhydryloxy und Triphenylmethoxy (Trityloxy), sowie Heterocyclyloxyreste, wie insbesondere 2-Tetrahydropyranyloxy. Als eine besondere veretherte Hydroxylgruppe ist die Gruppierung Methylendioxy und Ethylendioxy zu bezeichnen, die erstere substituiert in der Regel 2 benachbarte C-Atome, insbesondere in Arylresten, die zweite ist an einem und demselben C-Atom gebunden und dient als Schutzgruppe für Oxo.

Eine als Substituent im Hydrocarbyl vorliegende veresterte Hydroxylgruppe trägt einen Acylrest $Ac^o$ mit höchstens 12 C-Atomen, welcher auch innerhalb dieser Gesamtzahl der C-Atome analog wie der Rest $Ac^1$ substituiert sein kann, oder ist durch eine im Hydrocarbyl auch anwesende Carboxylgruppe lactonisiert.

Eine als Substituent im Hydrocarbyl vorliegende veresterte Carboxylgruppe ist eine solche, in welcher das Wasserstoffatom durch einen der oben charakterisierten Kohlenwasserstoffreste, vorzugsweise einen Niederalkyl- oder Phenylniederalkylrest, ersetzt ist; als Beispiel einer veresterten Carboxylgruppe sind insbesondere die Methoxy-, Ethoxy-, tert-Butoxy- und Benzyloxycarbonylgruppe, sowie auch eine lactonisierte Carboxylgruppe zu nennen.

Eine primäre Aminogruppe $-NH_2$ als Substituent des Hydrocarbyls kann auch in geschützter Form als eine dieser Gruppe entsprechende Acylaminogruppe der Formel $-NH-Ac^o$, worin $Ac^o$ die oben charakterisierte Bedeutung hat, vorliegen. Eine sekundäre Aminogruppe trägt anstelle eines der beiden Wasserstoffatome einen Hydrocarbylrest, vorzugsweise einen unsubstituierten, wie einen der obengenannten, und kann auch in einer geschützten Form als eine davon abgeleitete Acylaminogruppe mit einem nachstehend charakterisierten monovalenten Acylrest $Ac_a^o$ vorliegen.

Der als Amino-Schutzgruppe dienende Acylrest $Ac_a^o$ leitet sich vorzugsweise von einem Kohlensäurehalbderivat ab und ist vorzugsweise gegebenenfalls, insbesondere durch Niederalkyl, Niederalkoxy, Nitro und/oder Halogen, substituiertes Niederalkoxycarbonyl oder Arylniederalkoxycarbonyl, wie Methoxycarbonyl, Ethoxycarbonyl, tert-Butoxycarbonyl, 2,2,2-Trichlorethoxycarbonyl, 2-Iodethoxycarbonyl, Benzyloxycarbonyl, 2-Phenyl-2-propoxycarbonyl, 2-p-Tolyl-2-propoxycarbonyl, 2-(p-Biphenylyl)-2-propoxycarbonyl oder 9-Fluorenylmethoxycarbonyl.

Eine als Substituent im Hydrocarbyl vorkommende tertiäre Aminogruppe trägt 2 verschiedene oder, vorzugsweise, gleiche Hydrocarbylreste (einschliesslich der heterocyclischen Reste), wie die oben charakterisierten unsubstituierten Hydrocarbylreste.

Eine bevorzugte Aminogruppe ist eine solche der Formel $R^1\text{-}\overset{|}{N}\text{-}R^2$, worin $R^1$ und $R^2$ unabhängig voneinander je Wasserstoff, unsubstituiertes acyclisches $C_1$-$C_7$-Hydrocarbyl (wie insbesondere $C_1$-$C_4$-Alkyl oder $C_1$-$C_4$-Alkenyl) oder monocyclisches, gegebenenfalls durch $C_1$-$C_4$-Alkyl, $C_1$-$C_4$-Alkoxy, Halogen und/oder Nitro substituiertes Aryl, Aralkyl oder Aralkenyl mit höchstens 10 C-Atomen darstellen, wobei die kohlenstoffhaltigen Reste durch eine Kohlenstoff-Kohlenstoff-Bindung oder ein Sauerstoff-, Schwefel- oder gegebenenfalls durch Hydrocarbyl substituiertes Stickstoffatom untereinander gebunden sein können. In einem solchen Fall bilden sie zusammen mit dem Stickstoffatom der Aminogruppe einen stickstoffhaltigen heterocyclischen Ring. Als Beispiel von besonders bevorzugten freien Aminogruppen sind die folgenden zu nennen: Diniederalkylamino, wie Dimethylamino, Diethylamino, Pyrrolidino, Piperidino, Morpholino, Thiomorpholino und Piperazino oder 4-Methylpiperazino, gegebenenfalls durch Niederalkyl, Niederalkoxy, Halogen und/oder Nitro substituiertes Diphenylamino und Dibenzylamino; unter den geschützten dann insbesondere Niederalkoxycarbonylamino, wie tert-Butoxycarbonylamino, Phenylniederalkoxycarbonylamino, wie 4-Methoxybenzyloxycarbonylamino, sowie 9-Fluorenylmethoxycarbonylamino.

Ein bevorzugter Hydrocarbylrest $R^o$ im Acylrest $R^o$-C(=O)- ist z.B. $C_1$-$C_{19}$-Alkyl, insbesondere solches, das bei mehr als 5 C-Atomen eine lineare Kette aufweist und welches die folgenden Substituenten tragen kann: eine Carboxylgruppe, die gegebenenfalls auch in Salzform oder als eine Cyanogruppe oder ein $C_1$-$C_4$-Alkylester ($C_1$-$C_4$-Alkoxycarbonylgruppe) vorliegen kann und welche sich vorzugsweise in $\omega$-Stellung befindet, eine Aminogruppe der oben definierten Formle $R^1$-N-$R^2$, vorzugsweise eine solche, worin $R^1$ und $R^2$ je Wasserstoff ist und sich dann vorzugsweise in 1-Stellung befindet, oder ein oder mehrere Halogenatome, insbesondere Fluor oder Chlor, die sich vorzugsweise in der Nachbarschaft der Carbonylgruppe befinden. Ein anderes bevorzugtes Acyl $Ac_a^1$ ist ein bicyclisches oder insbesondere monocyclisches Aroyl, vor allem Benzoyl, welches auch einen oder mehrere der folgenden Substituenten tragen kann: Halogenatome, insbesondere Chlor oder Fluor, Nitrogruppen, $C_1$-$C_4$-Alkylreste, insbesondere Methyl, Hydroxylgruppen und veretherte Hydroxylgruppen, insbesondere $C_1$-$C_4$-Alkoxy, wie Methoxy, Phenoxy und Methylendioxy, sowie Carboxylgruppen, welche auch in der Salzform oder als eine Cyanogruppe oder ein $C_1$-$C_4$-Alkylester ($C_1$-$C_4$-Alkoxycarbonyl) vorliegen können. Vorzugsweise tragen die Aroylreste nicht mehr als 2, vor allem nur einen solchen Substituenten. Bevorzugt sind auch analoge Heteroaroylreste, insbesondere solche, die sich von Pyridin, Furan, Thiophen und Imidazol, und von ihren Analogen mit kondensiertem Benzoring (wie Chinolin, Isochinolin, Benzofuran und Benzimidazol) ableiten und gegebenenfalls auch, wie oben angegeben substituiert sind. Bevorzugte Acylreste dieser Art leiten sich auch vom Benzyl und Styryl ab (d.h. Phenacetyl und Cinnamoyl), sie können auch in der oben angegebenen Weise substituiert sein.

Derartige Acylreste $Ac_a^1$ bilden mit dem Amino-Stickstoff von Desferrioxamin B entsprechende Acylamide, unter welchen diejenigen mit den obengenannten Bedeutungen von $Ac_a^1$ besonders bevorzugt sind. Beispielsweise sind N-Acylderivate von Desferrioxamin B zu nennen, die sich von den folgenden Carbonsäuren ableiten: aliphatische Monocarbonsäuren mit höchstens 20 Kohlenstoffatomen, wie Niederalkancarbonsäuren, z.B. Propion-, Butter-, Isobutter-, Valerian-, Isovalerian, Capron-, Trimethylessig-, Oenanth- und Diethylessigsäure und vor allem Essigsäure, sowie Laurin-, Myristin-, Palmitin- und Stearinsäure, sowie Oelsäure, Elaidinsäure, Linolsäure und Linolensäure, aber auch entsprechende halogenierte Niederalkancarbonsäuren, wie Chloressigsäure, Bromessig-oder $\alpha$-Bromisovaleriansäure, carbocyclische oder carbocyclisch-acyclischeMonocarbonsäuren, z.B. die Cyclopropan-, Cyclopentan- und Cyclohexan-carbonsäure bzw. die Cyclopentan- oder Cyclohexan-essigsäure oder -propionsäure; aromatische carbocyclische Carbonsäuren, z.B. Benzoesäure, die einfach oder mehrfach, wie oben angegeben, substituiert sein kann; Aryl- oder Aryloxy-niederalkancarbonsäuren und deren in der Kette ungesättigte Analoga, z.B. gegebenenfalls, wie oben für die Benzoesäure angegeben, substituierte Phenylessig- bzw. Phenoxyessigsäuren, Phenylpropionsäuren und Zimtsäuren; und heterocyclische Säuren, z.B. Furan-2-carbonsäure, 5-tert-Butylfuran-2-carbonsäure, Thiophen-2-carbonsäure, Nicotin- oder Isonicotinsäure, 4-Pyridinpropionsäure, und gegebenenfalls durch Niederalkylreste substituierte Pyrrol-2-oder -3-carbonsäuren; ferner auch entsprechende $\alpha$-Aminosäuren, insbesondere die in der Natur vorkommenden $\alpha$-Aminosäuren der L-Reihe, z.B. Glycin, Phenylglycin, Prolin, Leucin, Valin, Tyrosin, Histidin und Asparagin, vorzugsweise in einer N-geschützten Form, d.h. in einer solchen, in welcher die Aminogruppe durch eine konventionelle, z.B. eine der obengenannten, Aminoschutzgruppe substituiert ist; weiter auch Dicarbonsäuren, wie Oxalsäure, Malonsäure, Mono- oder Di-niederalkylmalonsäuren, Bernsteinsäure, Glutarsäure, Adipinsäure, Erucasäure, Maleinsäure, eine durch Halogen, wie Fluor, Chlor oder Brom, und/oder Niederalkyl, Hydroxy, Niederalkoxy und Nitro gegebenenfalls substituierte Phthal-, Chinolin-, Isochinolin- oder Phenylbernsteinsäure, sowie auch Glutaminsäure und Asparaginsäure, wobei die zwei letztgenannten Säuren vorzugsweise mit geschützten Aminogruppen vorliegen. Wie bereits gesagt wurde, kann die zweite Carboxylgruppe nicht nur frei, sondern auch funktionell abgewandelt, z.B. als ein $C_1$-$C_4$-Alkylester oder als ein Salz, vorzugsweise als ein physiologisch verträgliches Salz mit einer salzbildenden basischen Komponente, vorhanden sein. In Betracht kommen in erster Linie Metall- oder Ammoniumsalze, wie Alkalimetall- und Erdalkalimetall-, z.B. Natrium-, Kalium-, Magnesium- oder Calciumsalze, bzw. Ammoniumsalze mit Ammoniak oder geeigneten organischen Aminen.

Ein von Monoestern der Kohlensäure abgeleiteter Acylrest $Ac_b^1$ ist durch die Teilformel $R^o$-O-CO- charakterisiert. Mit der Grundstruktur des Desferrioxamins B bildet dieses Acyl dann entsprechende N-mono-substituierte Urethane. Als Beispiel solcher Acylreste sind diejenigen zu nennen, worin $R^o$ die folgenden bevorzugten Bedeutungen eines acyclischen Hydrocarbylrestes darstellt: $C_1$-$C_{20}$-Alkyl, $C_2$-$C_{20}$-Hydroxyalkyl, dessen Hydroxylgruppe sich in beliebiger Stellung ausser 1-Stellung, vorzugsweise in 2-Stellung befindet, Cyano-[$C_1$-$C_{20}$]-alkyl, dessen Cyanogruppe sich vorzugsweise in 1- oder $\omega$-Stellung befindet, oder Carboxy-[$C_1$-$C_{20}$]-alkyl, dessen Carboxylgruppe sich vorzugsweise in 1- oder $\omega$-Stellung befindet und gegebenenfalls auch in Salzform oder als ein $C_1$-$C_4$-Alkylester ($C_1$-$C_4$-Alkoxycarbonyl) oder Benzylester (Benzyloxycarbonyl) vorliegen kann, sowie $C_1$-$C_{20}$-Alkenyl, dessen freie Valenz sich nicht an

demselben C-Atom wie die Doppelbindung befindet, wobei alle genannten Reste, ausgenommen diejenigen mit der $C_3$-$C_5$-Alkyl-Grundstruktur, eine lineare (unverzweigte) Alkylkette aufweisen; weiter auch lineares (Mono-, Di- bis Hexa)-oxaalkyl mit 4-20 Kettengliedern, worin eines oder mehrere der C-Atome, von C-3 an, eines linearen $C_4$-$C_{20}$-Alkyls durch Sauerstoffatome, welche voneinander durch mindestens 2 C-Atome getrennt sind und vorzugsweise sich in den Stellungen 3, 6, 9, 12, 15 und 18 befinden, ersetzt ist. Bevorzugt sind insbesondere Reste der Formel $-(CH_2-CH_2-O-)_n$niederalkyl, worin n für 1 bis 19 steht. Zu erwähnen sind auch Reste $Ac_b^1$ , worin $R^o$ die folgenden bevorzugten Bedeutungen eines carbocyclischen oder heterocyclischen, sowie auch carbocyclisch-acyclischen bzw. heterocyclisch-acyclischen Hydrocarbylrestes darstellt: bicyclisches oder vorzugsweise monocyclisches Aryl, vor allem Phenyl, welches einen oder mehrere der folgenden Substituenten tragen kann: Halogenatome, insbesondere Fluor und Chlor, $C_1$-$C_4$-Alkylreste, insbesondere Methyl, $C_1$-$C_4$-Alkoxygruppen, insbesondere Methoxy, Methylendioxy, Nitrogruppen und/oder Carboxylgruppen, welche frei, in einer Salzform, oder als $C_1$-$C_4$-Alkylester, insbesondere Methoxycarbonyl oder Ethoxycarbonyl, vorliegen können. Vorzugsweise tragen die Arylreste nicht mehr als 2 Substituenten, insbesondere solche gleicher Art, oder nur einen einzigen; vor allem sind sie unsubstituiert. Als bevorzugter heterocyclischer Hydrocarbyl- (Heterocyclyl-)rest kommt z.B. ein solcher in Betracht, welcher den oben hervorgehobenen Arylresten analog ist und anstelle eines oder zweier C-Atome je ein Heteroatom, insbesondere Stickstoff, enthält, wie ein Pyridyl oder Chinolyl, bzw. Chinazolyl, wobei die freie Valenz an einem C-Atom lokalisiert ist. Bevorzugte carbocyclisch-acyclische und heterocyclisch-acyclische Hydrocarbylreste sind solche, worin zwei oder drei, vorzugsweise aber nur einer, der oben definierten cyclischen Reste, vorzugsweise der unsubstituierten, durch $C_1$-$C_3$-Alkyl getragen wird, wobei sie vorzugsweise alle an einem C-Atom, vorzugsweise dem endständigen, lokalisiert sind; am meisten bevorzugt ist unsubstituiertes Benzyl.

Als besonders bevorzugte Hydrocarbylreste $R^o$ im Acyl $Ac_b^1$ sind beispielsweise die folgenden zu nennen: acyclisches Hydrocarbyl, insbesondere $C_1$-$C_{20}$-Alkyl, vorzugsweise lineares, welches durch eine Carboxylgruppe, vorzugsweise in funktionell abgewandelter Form, wie Salz, Cyano oder $C_1$-$C_4$-Alkylester, die sich vorzugsweise in der $\omega$-Stellung befindet, substituiert sein kann, oder ein analoges lineares (Mono bis Hexa)-oxaalkyl mit 4-20 Kettengliedern, insbesondere ein solches, das oben als besonders bevorzugt charakterisiert wurde. Bevorzugte Reste $R^o$ sind auch gegebenenfalls substituierte Phenyl- und Benzylreste, z.B. die oben als bevorzugt erwähnten.

Ein weiterer Acylrest leitet sich von Amiden der Kohlensäure ab und ist durch die Formel

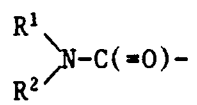

$(Ac_c^1)$

charakterisiert, worin $R^1$ und $R^2$ die obgenannten Bedeutungen haben. Mit dem Amino-Stickstoff von Desferrioxamin B bildet dieser Acylrest dann entsprechende Harnstoffe.

Unter bevorzugten Acylresten dieser Art sind insbesondere solche hervorzuheben, worin einer der Reste $R^1$ und $R^2$ Wasserstoff ist und der andere einen $C_1$-$C_7$-Alkylrest, vorzugsweise einen $C_1$-$C_5$-Alkylrest, bedeutet, welcher durch Hydroxyl, Mercapto, Methylthio, Phenyl, p-Hydroxyphenyl, p-Methoxyphenyl, 2-Indolyl, 2-Imidazolyl und vor allem durch eine oder mehrere Carboxylgruppen (frei oder in funktionell abgewandelter Form, wie $C_1$-$C_4$-Alkoxycarbonyl, Carbamoyl oder Amidino), wobei eine der freien oder funktionell abgewandelten Carboxylgruppen sich vorzugsweise in 1-Stellung befindet, substituiert sein kann. Dieser so substituierte $C_1$-$C_7$-Alkylrest entspricht vorzugsweise einem Radikal, dessen freie Valenz anstelle der Aminogruppe in einer geläufigen Aminosäure, wie $\beta$-Alanin, $\gamma$-Aminobuttersäure oder Norvalin, und insbesondere einer in der Natur als Peptid-Baustein vorkommenden $\alpha$-Aminosäure der L-Reihe, oder einem Antipoden davon, steht. Besonders bevorzugte Acylreste der Formel $Ac_c^1$ sind diejenigen, worin $R^1$ Wasserstoff und $R^2$ in 1-Stellung durch $C_1$-$C_4$-Alkoxycarbonyl substituiertes $C_1$-$C_5$-Alkyl bedeuten.

Zu erwähnen als ein besonderer Acylrest ist der Rest der Formel $Cl-SO_2-NH-CO-$ (Chlorsulfonylaminocarbonyl), welcher interessante Möglichkeiten zum weiteren Funktionalisieren bietet.

Ein weiterer organischer Acylrest Ac, bezeichnet als $Ac^2$, leitet sich von einer acyclischen, carbocyclischen, oder heterocyclischen, ferner auch einer carbocyclisch-acyclischen oder heterocyclisch-acyclischen Sulfonsäure ab und entspricht der Teilformel $R^o-O-SO_2-$ oder $R^o-SO_2-$, worin $R^o$ für Hydrocarbyl der oben erwähnten allgemeinen und, insbesondere, der bevorzugten Bedeutungen steht. Unter den erfindungsgemässen Verbindungen, die den Rest $Ac^2$ tragen, sind solche besonders hervorzuheben, worin $R^o$ $C_1$-$C_7$-Alkyl, insbesondere lineares $C_1$-$C_7$-Alkyl, bicyclisches oder monocyclisches Aryl, wie insbesondere Phenyl,

welches analog, wie oben für hervorgehobene Aroylreste geschildert wurde substituiert sein kann. Hervorzuheben sind auch analog gebaute bicyclische und monocyclische aromatische Heterocyclylreste, in welchen eines oder zwei der C-Atome durch Heteroatome ersetzt sind, wie 2- oder 4-Pyrimidyl, Chinolyl- oder Isochinolyl. Auch die Heterocyclylreste können Substituenten, insbesondere die für Aroyl hervorgehobenen, tragen (dabei ist z.B. ein Hydroxylderivat durch tautomere Verschiebung der Doppelbindung einem Dihydrooxo-Derivat gleich). Als eine besondere Ausgestaltung davon ist der Rest $O=C=N-SO_2$-(Isocyanatosulfonyl) zu erwähnen. Besonders bevorzugte Acylreste dieser Art sind N-(Niederalkoxysulfonyl)-carbamoyl- und Reste der Formel Niederalkoxy-$(CH_2-CH_2-O-)_n$CO-NH-$SO_2$-, worin n für 1-19 steht.

Ein noch weiterer organischer Acylrest Ac ist der von einer Phosphorsäure abgeleitete Acylrest $Ac^3$. Dieser ist z.B. ein von Pyrophosphorsäure oder vor allem von Orthophosphorsäure abgeleiteter Hydrocarbylester oder Amid. Von den erfindungsgemässen Acylgruppen $Ac^3$ sind insbesondere solche der Teilformel

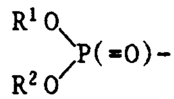

hervorzuheben, worin $R^1$ und $R^2$ die oben angegebenen allgemeinen und besonders hervorgehobenen Bedeutungen ausser Wasserstoff haben und vorzugsweise beide gleich sind und ein unsubstituiertes $C_1$-$C_7$-Alkyl, insbesondere ein lineares, wie vor allem Methyl oder Ethyl, oder aber ein gegebenenfalls, insbesondere durch $C_1$-$C_4$-Alkyl, $C_1$-$C_4$-Alkoxy, Halogen und/oder Nitro substituiertes Phenyl darstellen.

Vorzugsweise bedeutet der Acylrest Ac $C_2$-$C_{20}$-Alkanoyl, Benzoyl, $C_{18}$-Alkenoyl, ($C_1$-$C_{12}$-Alkoxy)-carbonyl, einen Rest der Formel -CO-O-$(CH_2-CH_2-O-)_n$niederalkyl, worin n für 1 bis 19 steht, Chlorcarbonyl, (1-Imidazolyl)-carbonyl, N-Diniederalkyl-carbamoyl, in 1- oder 2-Stellung des Alkylteiles durch ($C_1$-$C_4$-Alkoxy)-carbonyl substituiertes ($C_1$-$C_5$-Alkyl)-aminocarbonyl, N-(Chlorsulfonyl)-carbamoyl, N-(Niederalkoxysulfonyl)-carbamoyl oder einen Rest der Formel -$SO_2$-NH-CO$(-O-CH_2-CH_2)_n$niederalkoxy, worin n für 0 bis 19 steht.

Im allgemeinen werden die als Acylrest Ac Chlor-carbonyl, (1-Imidazolyl)-carbonyl oder N-(Chlorsulfonyl)-carbamoyl aufweisenden Reaktionsprodukte in einem weiteren Reaktionsschritt unter Chlor-Ersatz in andere Produkte der Formel I überführt, worin der Acylrest Ac vorzugsweise eine der übrigen obengenannten Bedeutungen hat.

Als erfindungsgemäss anzuwendende, den Acylrest Ac einführende Mittel werden die üblichen, zu diesem Zweck allgemein gebräuchlichen Acylierungsmittel verwendet; insbesondere verwendet man Acylierungsmittel der Formel AcY (III), worin Ac die oben, z.B. für $Ac^1$, $Ac^2$ und $Ac^3$, angegebenen allgemeinen und hervorgehobenen Bedeutungen hat, und Y eine reaktionsfähige funktionell abgewandelte Hydroxylgruppe ist oder eine zusätzliche Bindung im Rest Ac darstellt, die Wasserstoff an dem zur Carbonylgruppe $\alpha$-ständigen Atom ersetzt.

Ein Acylierungsmittel, das sich vom oben definierten Acylrest $Ac^1$ einer Carbonsäure ableitet, ist insbesondere ein solches der Formel

Z-X-C(=O)-Y (IIIA),

worin X und Z die oben angegebenen Bedeutungen haben und Y eine reaktionsfähige funktionell abgewandelte Hydroxylgruppe ist oder eine zusätzliche einfache Bindung darstellt, deren anderes Ende ein Wasserstoffatom entweder in der Iminogruppe (falls X für -NH- steht) oder am ersten C-Atom des Hydrocarbyls $R^o$ (falls X für eine einfache Bindung und Z für einen geeigneten Hydrocarbylrest $R^o$ steht) ersetzt.

Eine reaktionsfähige funktionell abgewandelte Hydroxylgruppe ist in erster Linie ein veresterte Hydroxylgruppe, beispielsweise eine solche, die mit einer starken anorganischen Säure, wie einer Halogenwasserstoffsäure, z.B. Chlor-, Brom- oder Iodwasserstoffsäure, einer Pseudohalogenwasserstoffsäure,wie Azoimid oder Imidazol (unter Abspaltung des H-Atoms von 1-N-Atom), einer sauerstoffhaltigen Mineralsäure, wie Phosphorsäure und insbesondere Schwefelsäure, oder einer starken organischen, wie aliphatischen oder aromatischen Sulfonsäure (z.B. Methan- und Ethan- bzw. Benzol-, p-Toluol-, p-Nitrobenzol- und p-Chlorbenzolsulfonsäure) verestert wird. Eine derartige veresterte Gruppe bildet dann mit dem Acylrest ein gemischtes Anhydrid. Darunter besonders hervorzuheben sind gemischte Anhydride mit Halogenwasserstoffsäuren und Pseudohalogenwasserstoffsäuren, wie Säurebromide, Säurechloride, Säureazide und 1-Imidazolyl-Derivate der Formel

Z-X-C(=O)-Hal,

worin Hal Brom oder Azido und vorzugsweise Chlor oder 1-Imidazolyl bedeutet und Z und X die oben genannten Bedeutungen haben. Als ein Reagens dieses Typs sind Phosgen und sein weniger toxisches Analogon Bis-(1-imidazolyl)-carbonyl (und ähnliche Reagentien) zu erwähnen. Diese setzt man in der Regel in äquimolaren Mengen ein, so dass die zweite reaktionsfähige Gruppe Y im Produkt erhalten bleibt und nachträglich abgewandelt werden kann.

Bevorzugte Säurechloride sind unter anderem ausgewählt aus $C_2$-$C_{20}$-Alkansäurechlorid, Benzoylchlorid, $C_{18}$-Alkensäurechlorid, Chlorameisensäure-($C_1$-$C_{12}$-alkyl)-ester, einem Chlorameisensäureester der Formel $Cl-CO-O-(CH_2-CH_2-O-)_n$niederalkyl, worin n für 1 bis 19 steht, sowie einem Chlorameisensäureester der Formel Cl-CO-NH-($C_1$-$C_5$-Alkylen)-COO-($C_1$-$C_4$-Alkyl).

Die reaktionsfähige veresterte Hydroxylgruppe kann aber auch entweder durch den Rest einer anderen Carbonsäure, insbesondere einer stärkeren Carbonsäure, wie der Ameisensäure, Chloressigsäure oder vornehmlich der Trifluoressigsäure, verestert werden und einem gemischten Anhydrid zugrundeliegen, oder aber durch denselben Acylrest verestert werden und ein symmetrisches Carbonsäureanhydrid der Formel $Ac^1$-O-$Ac^1$, insbesondere eines der Formeln $R^o$-CO-O-CO-$R^o$ oder $R^o$-O-CO-O-CO-O-$R^o$ bilden.

Eine bei Acylresten $Ac^2$ und $Ac^3$ vorteilhafte Bedeutung von Y ist eine reaktionsfähige, durch starke Säuren veresterte Hydroxylgruppe, wie die oben definierte, welche mit dem Acylrest ein gemischtes Säureanhydrid bildet. Darunter besonders hervorzuheben sind gemischte Anhydride mit Halogenwasserstoffsäuren, insbesondere mit Bromwasserstoffsäure und vor allem Chlorwasserstoffsäure, d.h. Säurebromide bzw. Säurechloride, z.B. diejenigen der Formeln

$R^O-SO_2$-Hal und 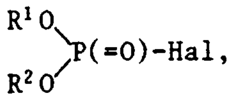

worin Hal Brom und vorzugsweise Chlor bedeutet und $R^o$, $R^1$ und $R^2$ die obengenannten Bedeutungen haben.

Acylierungsmittel der Formel IIIA, in welchen Y eine zusätzliche Bindung zum Rest $R^o$ oder -NH- darstellt, leiten sich insbesondere von Acylresten $Ac^1$ der Carbonsäuren ab, die am Nachbaratom zur Carbonylgruppe (d.h. am benachbarten Kohlenstoff- oder Stickstoffatom) Wasserstoff tragen; sie gehören zur Kategorie der Ketene bzw. Isocyanate und entsprechen den Formeln $R^o_a$ =C=O bzw. $R^o$-N=C=O, worin $R^o$ die oben definierte Bedeutung hat und $R^o_a$ für Hydrocarbyliden steht, d.h. für einen zweiwertigen, dem Rest $R^o$ entsprechenden Rest aliphatischen Charakters, in welchem das funktionalisierte Kohlenstoffatom durch einfache Bindungen mit benachbarten Kohlenstoff- und/oder Wasserstoffatomen verbunden ist. Als ein besonderes Mittel dieser Art ist Chlorsulfonylisocyanat ($ClSO_2$-N=C=O) zu erwähnen. Dieses wird in der Regel so eingesetzt, dass von beiden seiner reaktiven Gruppierungen nur eine, vorzugsweise die Isocyanatogruppe, reagiert; vorzugsweise acyliert man mit nur einem Aequivalent des Mittels.

Die erfindungsgemässe Umsetzung mit dem Acylierungsmittel der Formel III erfolgt unter Verfahrensbedingungen, die in der organischen Chemie allgemein für die Acylierung von Aminen gebräuchlich sind, üblicherweise bei Temperaturen zwischen dem Gefrierpunkt und dem Siedepunkt des Reaktionsgemisches, wie im Temperaturbereich von etwa -10 bis etwa +160°, insbesondere von etwa +20 bis etwa +50°, beim atmosphärischen oder erhöhten Druck, in heterogener Phase (wie Suspension) unter Rühren oder Umschütteln, oder vornehmlich in homogener flüssiger Phase, wie in einem Ueberschuss von flüssigem Reagens oder insbesondere in Anwesenheit von Lösungsmitteln, insbesondere organischen Lösungsmitteln, und gegebenenfalls in Gegenwart von säurebindenden anorganischen oder organischen Mitteln.

Geeignete Lösungsmittel sind beispielsweise aprotische organische Lösungsmittel niedriger Polarität, wie halogenierte, insbesondere chlorierte, aliphatische Kohlenwasserstoffe, wie Chloroform und Dichlormethan, und insbesondere polare aprotische Lösungsmittel, wie aliphatische und cyclische Ether, z.B. Diethylether, 1,2-Dimethoxyethan und Diisopropylether bzw. Dioxan und Tetrahydrofuran, niederaliphatische Ester und Amide, wie Ethylacetat bzw. Formamid, Acetamid, N,N-Dimethylacetamid und Dimethylformamid, sowie Acetonitril, Dimethylsulfoxid und Hexamethylphosphortriamid. Die Lösungsmittel können auch in zweckmässigen Kombinationen, z.B. zur Erhöhung der Löslichkeit von Komponenten, eingesetzt werden.

Als säurebindende Mittel können im Prinzip beliebige basische Verbindungen verwendet werden, wie einerseits organische stickstoffhaltige Basen, z.B. tertiäre Amine vom Typ Triethylamin, Ethyldiisopropylamin, N,N-Dimethylanilin, N-Ethylpiperidin oder N,N′-Dimethylpiperazin, oder aromatische heterocyclische Basen vom Typ Pyridin, Collidin, Chinolin oder 4-Dimethylaminopyridin, andererseits basisch reagierende anorganische Verbindungen, wie Alkalimetallhydroxide, -carbonate und -hydrocarbonate, sowie Salze von Carbonsäuren, wie Natrium- oder Kaliumacetat. Schliesslich können, diese Rolle auch neutral reagierende stickstoffhaltige Verbindungen übernehmen, die zugleich oft auch vorteilhafte Lösungsmittel darstellen, z.B. Carbonsäureamide, insbesondere niederaliphatische Carbonsäureamide, wie die obengenannten, und cyclische Amide, wie N-Methylpyrrolidon, sowie Amidoderivate der Kohlensäure, wie Urethane und Harnstoff. Umgekehrt können die oben erwähnten Basen, insbesondere die vom Typ Pyridin, als Lösungsmittel dienen.

Obwohl der Acylierungsreaktion immer dasselbe Prinzip zugrundeliegt und die Umsetzung nach einem einheitlichen Grundschema erfolgt, ist es für ein optimales Resultat notwendig, bei praktischer Durchführung die Eigenart der Reaktionskomponenten, in erster Linie die des jeweiligen Reaktionsmittels der Formel III, zu berücksichtigen.

Bei Acylierungen mit den oben geschilderten gemischten oder auch symmetrischen Anhydriden als Acylierungsmittel arbeitet man vorzugsweise in Anwesenheit eines säurebindenden Mittels, wie eines oben erwähnten, welches man vornehmlich in äquivalenter Menge oder einem kleineren Ueberschuss (der normalerweise 2 Aequivalente nicht übersteigt) einsetzt. Die Acylierung mit Isocyanaten kann, ihrer Natur nach, auch ohne säurebindende Mittel erfolgen, wobei ein Ausschluss von Feuchtigkeit und/oder protischen Lösungsmitteln empfohlen wird.

Falls der Hydrocarbylrest $R^o$ durch funktionelle Gruppen substituiert ist, die während der Acylierung mitreagieren könnten, wie freie Carboxyl-, Hydroxyl- und insbesondere Aminogruppen, werden diese vornehmlich vorübergehend geschützt, oder vorzugsweise im angewendeten Acylierungsmittel bereits in geschützter Form vorliegen, und nach erfolgter Acylierung von diesen Schutzgruppen befreit.

So z.B. gehört zu den gewöhnlichsten Methoden zum Schutz von Carboxylgruppen die Veresterung. Die Freisetzung einer veresterten Carboxylgruppe erfolgt im allgemeinen durch konventionelle Hydrolyse, vor allem unter Einwirkung von Basen (wie vornehmlich von Alkalimetallhydroxiden, -carbonaten oder -hydrogencarbonaten), oder aber, bei geeigneten Estern, wie solchen von tertiären Alkoholen (z.B. tert-Butylalkohol), durch Acidolyse, z.B. mittels Fluorwasserstoff oder Trifluoressigsäure. Ester mit Benzylalkoholen können auch durch konventionelle Hydrogenolyse abgespalten werden.

Die zum vorübergehenden Schutz von Hydroxylgruppen anzuwendenden Gruppen und Abspaltungsmethoden sind auch allgemein bekannt, z.B. aus der Synthese von Peptiden. Insbesondere schützt man Hydroxylgruppen in der Form von Estern mit Carbonsäuren, wie mit Niederalkansäuren oder mit Monoestern der Kohlensäure (z.B. Formiate oder Acetate einerseits oder tert-Butoxy- oder Benzyloxy-carbonate andererseits), oder aber in der Form vom Ethern, wie insbesondere solchen von tertiären Alkoholen (z.B. tert-Butylalkohol), oder auch in der Form von Acetalen (z.B. insbesondere als 2-Tetrahydropyranylether). Die ersteren Schutzgruppen werden üblicherweise analog wie veresterte Carboxylgruppen abgespalten; beide letzteren werden vornehmlich durch Acidolyse entfernt.

Die zum vorübergehenden Schutz von primären und sekundären Aminogruppen verwendbaren Schutzgruppen entsprechen denjenigen, die bei der Synthese von Peptiden eingehend untersucht wurden und die breiteste Anwendung finden. Vorzugsweise werden die eingangs angegebenen Amino-Schutzgruppen angewendet. Ihre Abspaltung, welche sich generell nach ihrer spezifischen Natur richtet, erfolgt unter allgemein bekannten Bedingungen der Solvolyse (insbesondere der basischen Hydrolyse oder Acidolyse) bzw. Hydrogenolyse.

Vornehmlich werden die allgemeinen Bedingungen der konventionellen Abspaltung der funktionell abgewandelten Gruppen so gewählt, dass weder die Bindung zwischen dem Acylrest und der Aminogruppe des Desferrioxamins B noch dessen Grundstruktur beeinträchtigt wird.

Die erfindungsgemäss als zweite Verfahrensstufe durchzuführende Entfernung der O-gebundenen organischen Silylgruppen erfolgt gemäss Verfahren, welche in der organischen Chemie zur Spaltung der Silicium-Sauerstoff-Bindung in Silylethern und Silylestern ausgearbeitet wurden und im allgemeinen Gebrauch sind, insbesondere durch Solvolyse. Vornehmlich kann man die Abspaltung unter üblichen Bedingungen der Alkoholyse bewirken, z.B. durch Behandeln mit einem Niederalkanol, insbesondere Methanol, vorzugsweise in Gegenwart eines sauren Katalysators, z.B. eines Säureadditionssalzes einer organischen Base, welche als Reaktionslösung verwendet wird (wie Pyridinhydrochlorid). Eine solchen Variante hat den Vorteil, dass dabei leicht flüchtige einfache Silylether (wie insbesondere Trimethylsilyl-methyl-ether) gebildet werden, welche aus dem Reaktionsgemisch mühelos durch Abdestillieren, meistens zusammen mit den Lösungsmitteln, entfernt werden können.

Die Erfindung betrifft auch diejenigen Ausführungsformen des erfindungsgemässen Verfahrens, nach denen man von einer auf irgendeiner Stufe des Verfahrens als Zwischenprodukt erhältlichen Verbindung

ausgeht und die fehlenden Schritte durchführt, und insbesondere solche, bei denen man das als Ausgangsstoff anzuwendende N,O-silylierte Derivat von Desferrioxamin B unter den Reaktionsbedingungen bildet. Insbesondere wird ein solches Derivat durch Behandeln von Desferrioxamin B oder eines Säureadditionssalzes davon (bzw. eines entsprechenden teilweise O-acylierten Analogen) mit einem geeigneten Silylierungsmittel (siehe weiter unten), insbesondere Trimethylchlorsilan oder Dimethyldichlorsilan, unter den weiter unten geschilderten Bedingungen gebildet und in demselben Reaktionsmedium mit dem Acylierungsmittel behandelt. Da die Behandlung mit den üblichen Silylierungsmitteln sowie auch den erfindungsgemässen Acylierungsmitteln vorteilhaft unter analogen Bedingungen, insbesondere in gleichen Lösungsmitteln, erfolgt, wobei die Reagentien beider Arten untereinander in der Regel nicht reagieren, besteht eine vorteilhafte Durchführung des Verfahrens darin, dass man die erfindungsgemässe Acylierung anschliessend an die Silylierungsreaktion (d.h. die Herstellung des Ausgangsstoffes in situ) in demselben Reaktionsmedium, gegebenenfalls in Anwesenheit eines überschüssigen Silyierungsmittels, vornimmt. Geeignete Lösungsmittel für diese Variante sind z.B. die oben erwähnten polaren aprotischen Lösungsmittel und/oder tertiären organischen Basen, beispielsweise Pyridin. - Vornehmlich kann man auch die anschliessende Abspaltung der O-gebundenen Silylgruppen (und zugleich auch die Zerstörung vom überschüssigen Silylierungs- und/oder Acylierungsmittel) in demselben Reaktionsmedium vornehmen, insbesondere dadurch, dass man zum Reaktionsgemisch nach erfolgter Silylierung und Acylierung ein Niederalkanol, wie vorzugsweise Methanol, zugibt. Auch hier sind als Reaktionsmedium die oben beschriebenen polaren aprotischen Lösungsmittel und insbesondere die tertiären organischen Basen geeignet. Die Alkoholyse wird dabei durch den katalytischen Einfluss einer starken Säure (vor allem Chlorwasserstoff), welche durch das Zersetzen des Silylierungs- bzw. Acylierungsreagens entsteht, weiter beschleunigt.

Die als Acylierungsmittel beim erfindungsgemässen Verfahren verwendeten Verbindungen der Formeln III, IIIA und IV sind bekannt oder nach allgemein bekannten Herstellungsmethoden der organischen Chemie routinemässig erhältlich.

Die als Ausgangsmaterial verwendeten N,O-silylierten Derivate von Desferrioxamin B, insbesondere die der oben definierten Formel II, sind neu und bilden auch einen Gegenstand der vorliegenden Erfindung. Bevorzugte Verbindungen der oben angegebenen Formel II sind diejenigen, worin B, $B_o^1$, $B_o^2$ und $B_o^3$ alle ein und dieselbe Bedeutung eines eingangs definierten organischen Silylrestes Sil haben, insbesondere solche, worin B für Tri-($C_1$-$C_6$-alkyl)-silyl, vor allem Trimethylsilyl oder auch Dimethylchlorsilyl, steht.

Diese Verbindungen können nach an sich bekannten, zur N- und O-Silylierung von organischen Verbindungen allgemein gebräuchlichen Verfahren erhalten werden. Das entsprechende Verfahren zu ihrer Herstellung, welches auch einen Gegenstand der vorliegenden Erfindung bildet, wird beispielsweise so durchgeführt, dass man Desferrioxamin B (auch in Form eines Salzes davon) oder ein teilweise O-acyliertes Derivat davon, insbesondere eine Verbindung der allgemeinen Formel

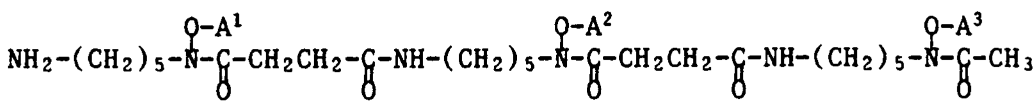

(V)

worin mindestens eines der Symbole $A^1$, $A^2$ und $A^3$ für Wasserstoff steht und die übrigen, jedes unabhängig von den anderen, Wasserstoff oder den Acylrest Ac mit der oben definierten Bedeutung darstellen, mit einem Silylierungsreagens der Formel Sil-Hal (VI), worin Sil die eingangs angegebenen Bedeutungen hat und Hal für Brom oder insbesondere Chlor steht, umsetzt.

Dabei werden auch hier Verbindungen mit den eingangs hervorgehobenen Bedeutungen der Symbole Sil und Ac bevorzugt, wie einerseits Desferrioxamin B der Formel V, worin $A^1$, $A^2$ und $A^3$ alle für Wasserstoff stehen, und andererseits ein Tri-($C_1$-$C_6$-Alkyl)silylchlorid, wor allem Trimethylsilylchlorid, oder Dimethyldichlorsilan als bevorzugte Reagentien der Formel VI. Wie bereits gesagt worden ist, kann Desferrioxamin B auch als Salz eingesetzt werden, insbesondere als ein Säureadditionssalz mit starken Säuren (z.B. den oben genannten), vor allem mit Chlorwasserstoff oder Methansulfonsäure, aus welchem der Grundstoff durch das basisch reagierende Reaktionsmedium freigesetzt wird.

Die Silylierung erfolgt unter den für diese Reaktion üblichen allgemeinen Bedingungen, z.B. bei Temperaturen zwischen dem Gefrierpunkt und dem Siedepunkt des Reaktionsgemisches, wie zwischen etwa -10° bis etwa +80°, vorzugsweise zwischen 0° und Raumtemperatur, vorzugsweise unter atmosphärischem Druck und in heterogener Phase (wie einer Suspension) unter Rühren oder Umschütteln, oder vornehmlich in homogener Phase in einem organischen Lösungsmittel und üblicherweise mit einem

Ueberschuss an Silylierungsmittel und in Gegenwart von säurebindenden anorganischen oder organischen Mitteln. - Geeignete Lösungsmittel sind beispielsweise aprotische organische Lösungsmittel niedrigerer Polarität, wie aliphatische und aromatische Kohlenwasserstoffe vom Typ Pentan, Hexan, Heptan und Cyclohexan bzw. Benzol, Toluol und Xylole, sowie halogenierte, insbesondere chlorierte, aliphatische Kohlenwasserstoffe, wie Chloroform und Dichlormethan, und insbesondere polare aprotische Lösungsmittel, wie aliphatische und cyclische Ether, z.B. Diethylether, 1,2-Dimethoxyethan und Diisopropylether bzw. Dioxan und Tetrahydrofuran, niederaliphatische Ester und Amide, wie Ethylacetat bzw. Formamid, Acetamid, N,N-Dimethylacetamid und Dimethylformamid, sowie Acetonitril, Dimethylsulfoxid und Hexamethylphosphorsäuretriamid; als das säurebindende Mittel verwendet man insbesondere organische stickstoffhaltige Basen, z.B. tertiäre Amine vom Typ Triethylamin, Ethyldiisopropylamin, N,N-Dimethylanilin, N-Ethylpiperidin oder N,N′-Dimethylpiperazin, oder aromatische heterocyclische Basen vom Typ Pyridin, Collidin, Chinolin oder 4-Dimethylaminopyridin.

Die Ausgangsstoffe der Formel II können, wie bereits erwähnt wurde, direkt und ohne vorherige Isolation aus dem Reaktionsgemisch für die N-Acylierung eingesetzt werden; gewünschtenfalls kann man sie aber durch geläufige Trennungsmethoden, wie Destillieren, Chromatographie und Kristallisation, in individueller Form erhalten.

Die Erfindung betrifft auch das mehrstufige Verfahren zur Herstellung der Verbindungen der Formel I, wobei man zunächst eine Verbindung der Formel V silyliert, die erhaltene Verbindung der Formel II acyliert und dann die noch vorhandenen Silylgruppen abspaltet.

Die nachfolgenden Beispiele illustrieren die vorliegende Erfindung, ohne sie in irgendeiner Weise einzuschränken. Temperaturen sind in Grad Celsius (°C) angegeben.

In den nachfolgenden Beispielen ist Carbowax die Schutzmarke der Firma Union Carbide, U.S.A., für Polyethylenglykol-monoether. Die Zahlenangabe in der näheren Bezeichnung, z.B. Carbowax MPEG 550, gibt das ungefähre durchschnittliche Molekulargewicht an. Carbowax MPEG 550 ist ein Gemisch von Polyethylenglykol-ω-monomethylethern, die im Durchschnitt 12 Einheiten der Formel $-CH_2-CH_2-O-$ aufweisen. Analog ist Carbowax MPEG 750 ein Gemisch von Polyethylenglykol-ω-monomethylethern mit durchschnittlich 16 Ethylenoxy-Einheiten.

Beispiel 1: N-Palmitoyl-desferrioxamin B

Eine Suspension von 66 g (100 mMol) Desferrioxamin B-Methansulfonat in 1000 ml Pyridin wird während 10 Min. mit 126 ml (1000 mMol) Trimethylchlorsilan (TMCS) versetzt und während 3 Std. bei Raumtemperatur gerührt, danch während 10 Minuten unterhalb 30°C mit 30 g (110 mMol) Palmitoylchlorid versetzt und weitere 19 Std. bei Raumtemperatur gerührt. Durch Zugabe von 300 ml Methanol zum Reaktionsgemisch lösen sich zuerst die Feststoffe, aber nach einigen Minuten beginnt sich die gewünschte Titelverbindung kristallin auszuscheiden.

Das kristalline Material wird aus 1000 ml Propanol/Wasser (1:1) umkristallisiert. Um restliche Palmitinsäure zu entfernen, wird das Kristallisat mit Diethylether digeriert, abfiltriert und im Hochvakuum getrocknet; Smp. 186-187°C.

Beispiel 2: N-[2-(2-Methoxyethoxy)-ethoxycarbonyl]-desferrioxamin B

Einer Suspension von 657 mg (1 mMol) Desferrioxamin B-Methansulfonat in 12 ml Pyridin wird 1,3 ml (10 mMol) Trimethylchlorsilan (TMCS) zugegeben, gefolgt nach 5 Min. bei 23°C unter Argon-Atmosphäre mit 201 mg (1,1 mMol) Chlorameisensäure-2-(2-methoxyethoxy)-ethylester in 1,0 ml Toluol. Das Reaktionsgemisch wird 3 Tage bei Raumtemperatur unter Argon gerührt. Ueberschüssige Reagentien werden bei der Zugabe von 10 ml Methanol zerstört und die Lösungsmittel abdestilliert. Der Rückstand wird im Hochvakuum getrocknet und aus Wasser kristallisiert; Smp. 142-144°.

Beispiel 3: N-[ω-Methoxy-(dodecakis-ethylenoxy)-carbonyl]-desferrioxamin B, worin die Bezeichnung "dodecakis-ethylenoxy" einen Rest der Formel $(CH_2-CH_2-O-)_n$ bedeutet, in welcher n für einen Durschnittswert von 12 steht.

Eine Suspension von 86,5 g (132 mMol) Desferrioxamin B-Methansulfonat in 2000 ml Pyridin versetzt man bei Raumtemperatur mit 194,0 ml (1500 mMol) Trimethylchlorsilan und rührt anschliessend 3 Std. bei Raumtemperatur. Zu diesem Reaktionsgemisch tropft man während 15 Min. bei Raumtemperatur das Acylierungsmittel, zubereitet durch Vermischen einer Lösung von 72,6 g (132 mMol) Carbowax MPEG 550 $[CH_3-O-(CH_2-CH_2-O-)_nH$, wobei n für einen Durchschnittswert von 12 steht] in 1000 ml Toluol mit 66,0 ml

(132 mMol) 20%iger Lösung von Phosgen in Toluol bei 70 ° C, dreistündiges Rühren bei dieser Temperatur und Abkühlen. Das Gemisch wird über Nacht bei Raumtemperatur gerührt, durch Zugabe von 2000 ml Methanol werden überschüssige Reagentien zerstört und Silylgruppen abgespalten, wonach die Lösungsmittel möglichst vollständig abdestilliert werden. Der Rückstand, noch stark pyridinhaltig, wird aus ca. 500 ml Methylenchlorid und 1000 ml Diethylether kristallisiert und über Nacht im Hochvakuum getrocknet. Durch Chromatographie an Sephadex® LH20 wird aus dem rohen Kristallisat die individuelle Titelverbindung mit 12 sich wiederholenden Ethylenoxygruppen erhalten; Smp. 131-132 ° nach Kristallisation aus Ethylacetat mit wenig Methylenchlorid.

Beispiel 4: N-[ω-Methoxy-(heptadecakis-ethylenoxy)-carbonyl]-desferrioxamin B der Formel I, worin die Bezeichnung "heptadecakis-ethylenoxy" einen Rest der Formel $(CH_2-CH_2-O-)_n$ bedeutet, in welcher n für einen Durchschnittswert von 17 steht.

Unter denselben Bedingungen und mit denselben Mengen der Komponenten wie in Beispiel 3 wird Desferrioxamin B-Methansulfonat silyliert und anschliessend mit einem Acylierungsreagens behandelt, welches aus 99,0 g (132 mMol) Carbowax MPEG 750 $[CH_3-O-(CH_2-CH_2-O)_nH$, worin n für einen Durchschnittswert von 17 steht] in 1000 ml Toluol und 66,0 ml (132 mMol) 20%igem Phosgen in Toluol analog Beispiel 3 zubereitet wird. Die in Beispiel 3 beschriebenen Bearbeitung ergibt die Titelverbindung, Smp. 125-126 ° .

Beispiel 5: N-[ω-Methoxy-(dodecakis-ethylenoxy)-carbonyl]-desferrioxamin B, worin die Bezeichnung"dodecakis-ethylenoxy" einen Rest der Formel $(CH_2-CH_2-O-)_n$ bedeutet, in welcher n für einen Durchschnittswert von 12 steht, hergestellt mit einem anderen Reagens.

In analoger Weise wie im Beispiel 3 werden 6,56 g (10 mMol) Desferrioxamin B-Methansulfonat in 150 ml Pyridin mit 15,5 mol (120 mMol) Trimethylchlorsilan silyliert und mit einem folgendermassen zubereiteten Acylierungsreagens umgesetzt: Eine Lösung von 5,5 g (10 mMol) Carbowax MPEG 550 in 50 ml Toluol wird mit 1,78 g (11 Mmol) Di-(1-imidazolyl)-carbonyl versetzt, bei 70 ° C 1 Stunde gerührt und abgekühlt. - Die Aufarbeitung gemäss Beispiel 3 gibt die Titelverbindung, welche mit derjenigen vom Beispiel 3 identisch ist.

Beispiel 6: N-(Ethoxycarbonylmethylcarbamoyl)-desferrioxamin B der Formel I, worin Ac = $C_2H_5-O-CO-CH_2-NH-CO-$ und $A^1 = A^2 = A^3 = H$.

In analoger Weise wie in Beispiel 1 werden 26,3 g (40 mMol) Desferrioxamin B-Methansulfonat in 300 ml Pyridin mit 50,0 ml (400 mMol) Trimethylchlorsilan (TMCS) silyliert. Nach 2,5 Std. wird die Reaktionslösung bei 22 ° C während 10 Min. mit 9,3 g (72 mMol) Isocyanatoessigsäure-ethylester versetzt und weitere 6 Std. bei Raumtemperatur gerührt. Durch Zugabe von 150 ml Methanol werden überschüssige Reagentien zerstört und die Silylgruppen abgespalten. Die Lösungsmittel werden durch Destillation entfernt. Der feste Rückstand wird im Hochvakuum getrocknet und zuerst aus Wasser, dann aus Methanol/Dichlormethan kristallisiert; Smp. 177-178 ° C.

Beispiel 7: N-(Methoxysulfonylcarbamoyl)-desferrioxamin B

In analoger Weise wie in Beispiel 1 werden 6,56 g (10 mMol) Desferrioxamin B-Methansulfonat in 150 ml Pyridin mit 15,5 ml (120 mMol) Trimethylchlorsilan silyliert und nach 1 Std. bei Raumtemperatur mit 0,95 ml (11 mMol) Chlorsulfonylisocyanat versetzt und 3 Std. bei Raumtemperatur gerührt. Durch Zugabe von Methanol werden überschüssige Reagentien zerstört, die Silylgruppen abgespalten und die Chlorsulfonylgruppe in die Methoxysulfonylgruppe umgewandelt. Das Reaktionsgemisch wird zur Trockne eingeengt. Der Rückstand wird im Hochvakuum getrocknet und durch Chromatographie an Sephadex® LH 20 gereinigt.

Das Produkt ist einheitlich gemäss Hochdruck-Flüssigkeitschromatographie (HPLC = "high-pressure liquid chromatography") unter den folgenden Bedingungen: Säule (4,0 x 120 mm) Hypersil® ODS; Lösungsmittel: A - 5millimolarer Phosphatpuffer vom pH = 3,0 B - 20:80-Gemisch (v/v) von Puffer A und Acetonitril; Gradient (Minuten/Lösung A:Lösung B): 0/100:0; 10/60:40; 12/0:100; 14/100:0; 15/100:0; Durchflussgeschwindigkeit: 2,3 ml/Minute.

Unter diesen Bedingungen ist die Retentionszeit RT = 7,13 Min. gegenüber RT = 8,50 Min. für Desferrioxamin B als Standard.

Beispiel 8: N-[ω-Methoxy-(dodecakis-ethylenoxy)-carbonylaminosulfonyl]-desferrioxamin B, worin die Bezeichnung "dodecakis-ethylenoxy" einen Rest der Formel $(CH_2-CH_2-O-)_n$ bedeutet, in welcher n für einen Durchschnittswert von 12 steht.

In analoger Weise und mit denselben Substanzmengen wie in Beispiel 5 wird Desferrioxamin B-Methansulfonat silyliert und anschliessend mit einem folgendermassen zubereiteten Acylierungsreagens umgesetzt:

Eine Lösung von 5,5 g (10 mMol) Carbowax MPEG 550 $[CH_3-O-(CH_2-CH_2-O-)_nH$, worin n für einen Durchschnittswert von 12 steht] in 50 ml Toluol wird mit 0,95 ml (11 mMol) Chlorsulfonylisocyanat versetzt, bei 70 °C 1 Std. gerührt und abgekühlt. Nach Zugabe von 2000 ml Methanol wird das Reaktionsgemisch zur Trockne eingeengt. Der Rückstand (stark pyridinhaltig) wird aus ca. 500 ml Methylenchlorid und 1000 ml Diethylether kristallisiert und über Nacht im Hochvakuum getrocknet. Eine weitere Reinigung kann durch Chromatographie an Sephadex® LH 20 erfolgen.

Unter den im Beispiel 7 angegebenen Bedingungen ist das Produkt in der HPLC einheitlich; RT = 10,25 Minuten gegenüber RT = 8,50 Min. für Desferrioxamin B als Standard.

**Patentansprüche**
**Patentansprüche für folgende Vertragsstaaten : AT, BE, CH, DE, FR, GB, GR, IT, LI, LU, NL, SE**

1. Ein Verfahren zur Einführung eines organischen Acylrestes selektiv an das Stickstoffatom der terminalen Aminogruppe von Desferrioxamin B oder eines teilweise O-acylierten Derivats davon, dadurch gekennzeichnet, dass man eine Verbindung der Formel II, worin

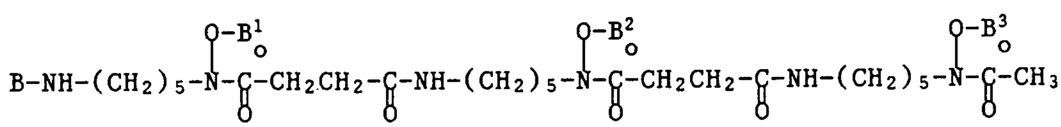

(II)

B eine organische Silylgruppe (Sil) der Formel

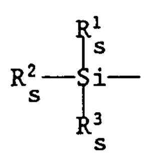 (Sil)

ist, in welcher $R_s^1$ und $R_s^2$ unabhängig voneinander je unsubstituiertes $C_{1-8}$-Hydrocarbyl und $R_s^3$ unsubstituiertes $C_{1-8}$-Hydrocarbyl oder Chlor bedeuten, und
mindestens eines der Symbole $B_o^1$ , $B_o^2$ und $B_o^3$ für eine organische Silylgruppe Sil steht und die übrigen, jedes unabhängig von anderen, für eine organische Silylgruppe Sil oder für einen organischen Acylrest Ac stehen,
mit einem einen Acylrest Ac einführenden Mittel umsetzt und die vorhandenen O-gebundenen organischen Silylgruppen Sil solvolytisch abspaltet.

2. Verfahren gemäss Anspruch 1, dadurch gekennzeichnet, dass man von Ausgangsstoffen der Formel II ausgeht, worin B, $B_o^1$ , $B_o^2$ und $B_o^3$ jeweils die gleiche Bedeutung haben und jeweils für Dimethylchlorsilyl oder Trimethylsilyl stehen.

3. Verfahren gemäss Anspruch 1, dadurch gekennzeichnet, dass man mit einem Acylierungsmittel der Formel AcY umsetzt, in welchem Ac für einen organischen Acylrest steht, der sich von einer gegebenenfalls funktionell abgewandelten Carbonsäure, einer organischen Sulfonsäure oder einer veresterten Phosphorsäure ableitet, und Y eine reaktionsfähige funktionell abgewandelte Hydroxylgruppe ist oder eine zusätzliche Bindung im Rest Ac darstellt, die Wasserstoff an dem zur Carbonylgruppe α-ständigen Atom ersetzt.

**4.** Verfahren gemäss Anspruch 3, dadurch gekennzeichnet, dass man mit einem Acylierungsmittel der Formel AcY umsetzt, in welcher Ac der Teilformel Z-X-C(=O)- entspricht, worin X eine einfache Bindung, Oxy oder gegebenenfalls substituiertes Imino und Z Hydrocarbyl $R^o$ oder, falls X eine einfache Bindung ist, auch Wasserstoff, Chlor oder 1-Imidazolyl darstellt.

**5.** Verfahren gemäss Anspruch 3, dadurch gekennzeichnet, dass man mit einem Acylierungsmittel der Formel AcY umsetzt, in welchem Ac durch die Teilformel $R^o$-O-CO- charakterisiert ist, worin $R^o$ $C_1$-$C_{20}$-Alkyl oder einen Rest der Formel $-(CH_2-CH_2-O-)_n$niederalkyl, worin n für 1 bis 19 steht, bedeutet.

**6.** Verfahren gemäss Anspruch 3, dadurch gekennzeichnet, dass man mit einem Acylierungsmittel der Formel AcY umsetzt, in welchem Ac durch die Formel

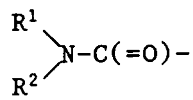

charakterisiert ist, worin $R^1$ und $R^2$ unabhängig voneinander je Wasserstoff, unsubstituiertes acyclisches $C_1$-$C_7$-Hydrocarbyl oder monocyclisches, gegebenenfalls durch $C_1$-$C_4$-Alkyl, $C_1$-$C_4$-Alkoxy, Halogen und/oder Nitro substituiertes Aryl, Aralkyl oder Aralkenyl mit höchstens 10 C-Atomen darstellen, oder worin $R^1$ Wasserstoff und $R^2$ in 1-Stellung durch $C_1$-$C_4$-Alkoxycarbonyl substituiertes $C_1$-$C_5$-Alkyl bedeuten.

**7.** Verfahren gemäss Anspruch 3, dadurch gekennzeichnet, dass man mit einem Acylierungsmittel der Formel AcY umsetzt, in welchem Ac den Rest $O=C=N-SO_2$- oder einen Rest der Formel Niederalkoxy-$(CH_2-CH_2-O-)_n$CO-NH-$SO_2$- bedeutet, worin n für 1-19 steht, und Y Brom oder Chlor ist.

**8.** Verfahren gemäss Anspruch 3, dadurch gekennzeichnet, dass man mit einem Acylierungsmittel der Formel $R^o$-N=C=O umsetzt, worin $R^o$ für unsubstituiertes oder in 1-Stellung durch $C_1$-$C_4$-Alkoxycarbonyl substituiertes $C_1$-$C_5$-Alkyl steht.

**9.** Verfahren gemäss Anspruch 3, dadurch gekennzeichnet, dass man mit Chlorsulfonylisocyanat umsetzt.

**10.** Verfahren gemäss Anspruch 3, dadurch gekennzeichnet, dass man mit Phosgen oder Bis-(1-imidazolyl)-carbonyl umsetzt.

**11.** Verfahren gemäss Anspruch 3, dadurch gekennzeichnet, dass man mit einem Säurechlorid ausgewählt aus $C_2$-$C_{20}$-Alkansäurechlorid, Benzoylchlorid, $C_{18}$-Alkensäurechlorid, Chlorameisensäure-($C_1$-$C_{12}$-alkyl)-ester, einem Chlorameisensäureester der Formel Cl-CO-O-$(CH_2-CH_2-O-)_n$niederalkyl, worin n für 1 bis 19 steht, sowie einem Chlorameisensäureester der Formel Cl-CO-NH-($C_1$-$C_5$-Alkylen)-COO-($C_1$-$C_4$-Alkyl), umsetzt.

**12.** Verfahren gemäss Anspruch 1, dadurch gekennzeichnet, dass man die Ausgangsstoffe so wählt, dass man eine Verbindung der Formel I,

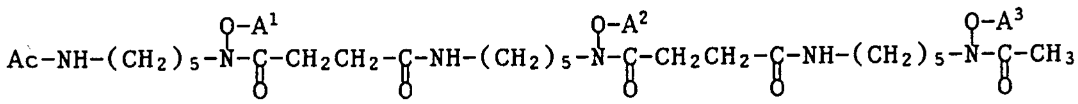

(I)

herstellt, worin Ac $C_2$-$C_{20}$-Alkanoyl, Benzoyl, $C_{18}$-Alkenoyl, ($C_1$-$C_{12}$-Alkoxy)-carbonyl, einen Rest der Formel -CO-O-$(CH_2-CH_2-O-)_n$niederalkyl, worin n für 1 bis 19 steht, Chlor-carbonyl, (1-Imidazolyl)-carbonyl, N-Diniederalkyl-carbamoyl, in 1- oder 2-Stellung des Alkylteiles durch ($C_1$-$C_4$-Alkoxy)-carbonyl substituiertes N-($C_1$-$C_5$-Alkyl)-carbamoyl, N-(Chlorsulfonyl)-carbamoyl, N-(Niederalkoxysulfonyl)-carbamoyl oder einen Rest der Formel $-SO_2-NH-CO(-O-CH_2-CH_2)_n$niederalkoxy, worin n für 0 bis 19 steht, und $A^1$, $A^2$ und $A^3$ je Wasserstoff bedeuten.

**13.** Eine Verbindung der Formel II, worin

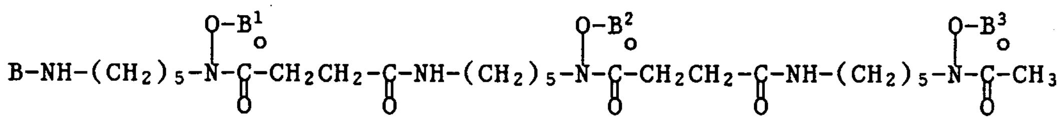

(II)

B, $B_o^1$ , $B_o^2$ und $B_o^3$ je für ein und dieselbe organische Silylgruppe (Sil) der Formel

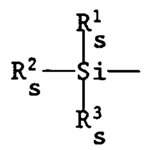

(Sil)

stehen, in welcher $R_s^1$ und $R_s^2$ je unsubstituiertes $C_1$-$C_8$-Hydrocarbyl und $R_s^3$ unsubstituiertes $C_1$-$C_8$-Hydrocarbyl oder Chlor bedeuten.

**14.** Eine Verbindung gemäss Anspruch 13, worin Sil für Dimethylchlorsilyl steht.

**15.** Eine Verbindung gemäss Anspruch 13, worin Sil für Trimethylsilyl steht.

**16.** Verwendung einer Verbindung gemäss einem der Ansprüche 13-15 als Ausgangsstoff für das Verfahren gemäss einem der Ansprüche 1-12.

**Patentansprüche für folgenden Vertragsstaat : ES**

**1.** Ein Verfahren zur Einführung eines organischen Acylrestes selektiv an das Stickstoffatom der terminalen Aminogruppe von Desferrioxamin B oder eines teilweise O-acylierten Derivats davon, dadurch gekennzeichnet, dass man eine Verbindung der Formel II, worin

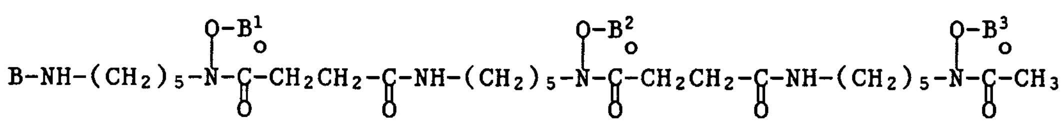

(II)

B eine organische Silylgruppe (Sil) der Formel

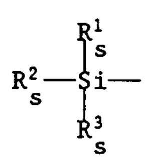

(Sil)

ist, in welcher $R_s^1$ und $R_s^2$ unabhängig voneinander je unsubstituiertes $C_{1-8}$-Hydrocarbyl und $R_s^3$ unsubstituiertes $C_{1-8}$-Hydrocarbyl oder Chlor bedeuten, und
mindestens eines der Symbole $B_o^1$ , $B_o^2$ und $B_o^3$ für eine organische Silylgruppe Sil steht und die übrigen, jedes unabhängig von anderen, für eine organische Silylgruppe Sil oder für einen organischen Acylrest Ac stehen,
mit einem einen Acylrest Ac einführenden Mittel umsetzt und die vorhandenen O-gebundenen organischen Silylgruppen Sil solvolytisch abspaltet.

**2.** Verfahren gemäss Anspruch 1, dadurch gekennzeichnet, dass man von Ausgangsstoffen der Formel II ausgeht, worin B, $B_o^1$ , $B_o^2$ und $B_o^3$ jeweils die gleiche Bedeutung haben und jeweils für Dimethylchlorsilyl oder Trimethylsilyl stehen.

**3.** Verfahren gemäss Anspruch 1, dadurch gekennzeichnet, dass man mit einem Acylierungsmittel der Formel AcY umsetzt, in welchem Ac für einen organischen Acylrest steht, der sich von einer gegebenenfalls funktionell abgewandelten Carbonsäure, einer organischen Sulfonsäure oder einer veresterten Phosphorsäure ableitet, und Y eine reaktionsfähige funktionell abgewandelte Hydroxylgruppe ist oder eine zusätzliche Bindung im Rest Ac darstellt, die Wasserstoff an dem zur Carbonylgruppe $\alpha$-ständigen Atom ersetzt.

**4.** Verfahren gemäss Anspruch 3, dadurch gekennzeichnet, dass man mit einem Acylierungsmittel der Formel AcY umsetzt, in welcher Ac der Teilformel Z-X-C(=O)- entspricht, worin X eine einfache Bindung, Oxy oder gegebenenfalls substituiertes Imino und Z Hydrocarbyl $R^o$ oder, falls X eine einfache Bindung ist, auch Wasserstoff, Chlor oder 1-Imidazolyl darstellt.

**5.** Verfahren gemäss Anspruch 3, dadurch gekennzeichnet, dass man mit einem Acylierungsmittel der Formel AcY umsetzt, in welchem Ac durch die Teilformel $R^o$-O-CO- charakterisiert ist, worin $R^o$ $C_1$-$C_{20}$-Alkyl oder einen Rest der Formel $-(CH_2-CH_2-O-)_n$niederalkyl, worin n für 1 bis 19 steht, bedeutet.

**6.** Verfahren gemäss Anspruch 3, dadurch gekennzeichnet, dass man mit einem Acylierungsmittel der Formel AcY umsetzt, in welchem Ac durch die Formel

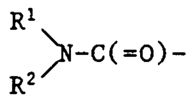

charakterisiert ist, worin $R^1$ und $R^2$ unabhängig voneinander je Wasserstoff, unsubstituiertes acyclisches $C_1$-$C_7$-Hydrocarbyl oder monocyclisches, gegebenenfalls durch $C_1$-$C_4$-Alkyl, $C_1$-$C_4$-Alkoxy, Halogen und/oder Nitro substituiertes Aryl, Aralkyl oder Aralkenyl mit höchstens 10 C-Atomen darstellen, oder worin $R^1$ Wasserstoff und $R^2$ in 1-Stellung durch $C_1$-$C_4$-Alkoxycarbonyl substituiertes $C_1$-$C_5$-Alkyl bedeuten.

**7.** Verfahren gemäss Anspruch 3, dadurch gekennzeichnet, dass man mit einem Acylierungsmittel der Formel AcY umsetzt, in welchem Ac den Rest $O=C=N-SO_2$- oder einen Rest der Formel Niederalkoxy-$(CH_2-CH_2-O-)_n$CO-NH-$SO_2$- bedeutet, worin n für 1-19 steht, und Y Brom oder Chlor ist.

**8.** Verfahren gemäss Anspruch 3, dadurch gekennzeichnet, dass man mit einem Acylierungsmittel der Formel $R^o$-N=C=O umsetzt, worin $R^o$ für unsubstituiertes oder in 1-Stellung durch $C_1$-$C_4$-Alkoxycarbonyl substituiertes $C_1$-$C_5$-Alkyl steht.

**9.** Verfahren gemäss Anspruch 3, dadurch gekennzeichnet, dass man mit Chlorsulfonylisocyanat umsetzt.

**10.** Verfahren gemäss Anspruch 3, dadurch gekennzeichnet, dass man mit Phosgen oder Bis-(1-imidazolyl)-carbonyl umsetzt.

**11.** Verfahren gemäss Anspruch 3, dadurch gekennzeichnet, dass man mit einem Säurechlorid ausgewählt aus $C_2$-$C_{20}$-Alkansäurechlorid, Benzoylchlorid, $C_{18}$-Alkensäurechlorid, Chlorameisensäure-($C_1$-$C_{12}$-alkyl)-ester, einem Chlorameisensäureester der Formel Cl-CO-O-$(CH_2-CH_2-O-)_n$niederalkyl, worin n für 1 bis 19 steht, sowie einem Chlorameisensäureester der Formel Cl-CO-NH-($C_1$-$C_5$-Alkylen)-COO-($C_1$-$C_4$-Alkyl), umsetzt.

**12.** Verfahren gemäss Anspruch 1, dadurch gekennzeichnet, dass man die Ausgangsstoffe so wählt, dass man eine Verbindung der Formel I,

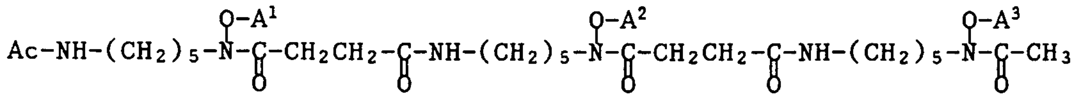

(I)

herstellt, worin Ac $C_2$-$C_{20}$-Alkanoyl, Benzoyl, $C_{18}$-Alkenoyl, ($C_1$-$C_{12}$-Alkoxy)-carbonyl, einen Rest der Formel -CO-O-($CH_2$-$CH_2$-O-)$_n$niederalkyl. worin n für 1 bis 19 steht, Chlor-carbonyl, (1-Imidazolyl)-carbonyl, N-Diniederalkyl-carbamoyl, in 1- oder 2-Stellung des Alkylteiles durch ($C_1$-$C_4$-Alkoxy)-carbonyl substituiertes N-($C_1$-$C_5$-Alkyl)-carbamoyl, N-(Chlorsulfonyl)-carbamoyl, N-(Niederalkoxysulfonyl)-carbamoyl oder einen Rest der Formel -$SO_2$-NH-CO(-O-$CH_2$-$CH_2$)$_n$niederalkoxy, worin n für 0 bis 19 steht, und $A^1$, $A^2$ und $A^3$ je Wasserstoff bedeuten.

**13.** Verfahren nach Anspruch 1, dadurch gekennzeichnet, dass man einen Ausgangsstoff der Formel II, worin

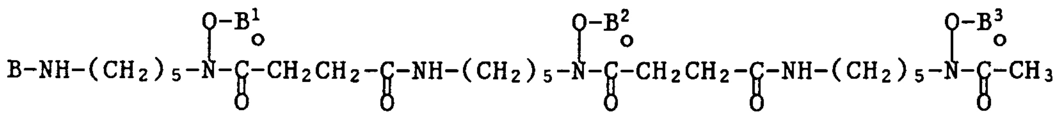

(II)

B eine organische Silylgruppe (Sil) der Formel

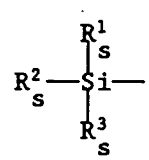

(Sil)

in welcher $R_s^1$ und $R_s^2$ unabhängig voneinander je unsubstituiertes $C_{1-8}$-Hydrocarbyl und $R_s^3$ unsubstituiertes $C_{1-8}$-Hydrocarbyl oder Chlor bedeutet, ist und mindestens eines der Symbole $B_o^1$ , $B_o^2$ und $B_o^3$ für Sil steht und die übrigen, jedes unabhängig von anderen, für Sil oder für einen organischen Acylrest Ac stehen, durch Umsetzung einer Verbindung der Formel

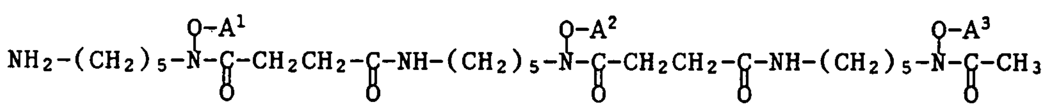

(V)

worin mindestens eines der Symbole $A^1$, $A^2$ und $A^3$ für Wasserstoff steht und die übrigen, jedes unabhängig von den anderen, Wasserstoff oder einen organischen Acylrest Ac darstellen, oder eines Säureadditonssalzes davon, mit einem Silylierungsreagens der Formel Sil-Hal (VI), worin Sil die oben angegebenen Bedeutungen hat und Hal für Brom oder Chlor steht, herstellt.

**14.** Verfahren gemäss Anspruch 13, dadurch gekennzeichnet, dass man Desferrioxamin B der Formel V, worin $A^1$, $A^2$ und $A^3$ alle für Wasserstoff stehen, oder ein Säureadditonssalz davon, mit einem Triniederalkylchlorsilan oder Diniederalkyldichlorsilan, umsetzt.

**15.** Verfahren gemäss Anspruch 13, dadurch gekennzeichnet, dass man Desferrioxamin B der Formel V, worin $A^1$, $A^2$ und $A^3$ alle für Wasserstoff stehen, oder ein Säureadditonssalz davon, mit Trimethylsilylchlorid, oder Dimethyldichlorsilan umsetzt.

**Claims**

**Claims for the following Contracting States : AT, BE, CH, DE, FR, GB, GR, IT, LI, LU, NL, SE**

1. A process for the introduction of an organic acyl radical selectively at the nitrogen atom of the terminal amino group of desferrioxamine B or of a partially O-acylated derivative thereof, which comprises reacting a compound of formula II

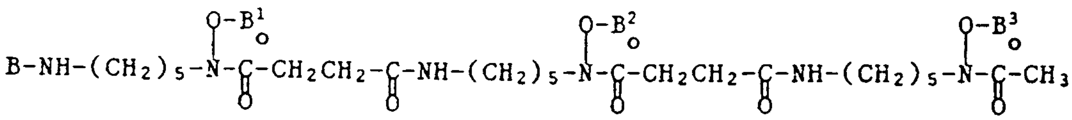

(II)

in which B is an organic silyl group (Sil) of formula

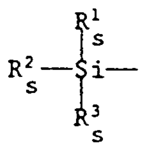

(Sil)

wherein $R_s^1$ and $R_s^2$ , independently of one another, are each unsubstituted $C_{1-8}$hydrocarbyl and $R_s^3$ is unsubstituted $C_{1-8}$hydrocarbyl or chlorine, and at least one of the symbols $B_o^1$ , $B_o^2$ and $B_o^3$ is an organic silyl group Sil and the others, independently of one another, are each an organic silyl group Sil or an organic acyl radical Ac, with an agent that introduces an acyl radical Ac, and removing the O-bonded organic silyl groups Sil present by means of solvolysis.

2. A process according to claim 1 which comprises using starting materials of formula II in which B, $B_o^1$ , $B_o^2$ and $B_o^3$ all have the same meaning and are each dimethylchlorosilyl or trimethylsilyl.

3. A process according to claim 1 which comprises reaction with an acylating agent of the formula AcY in which Ac is an organic acyl radical that is derived from an optionally functionally modified carboxylic acid, an organic sulfonic acid or an esterified phosphoric acid, and Y is a reactive functionally modified hydroxy group or is an additional bond in the Ac radical that replaces the hydrogen at the atom in the $\alpha$-position to the carbonyl group.

4. A process according to claim 3 which comprises reaction with an acylating agent of the formula AcY in which Ac corresponds to the partial formula Z-X-C(=O)- in which X is a single bond, oxy or unsubstituted or substituted imino and Z is hydrocarbyl $R^o$ or, if X is a single bond, also hydrogen, chlorine or 1-imidazolyl.

5. A process according to claim 3 which comprises reaction with an acylating agent of the formula AcY in which Ac is characterised by the partial formula $R^o$-O-CO- wherein $R^o$ is $C_1$-$C_{20}$alkyl or a radical of the formula -$(CH_2$-$CH_2$-O-$)_n$-lower alkyl in which n is from 1 to 19.

6. A process according to claim 3 which comprises reaction with an acylating agent of the formula AcY in which Ac is characterised by the formula

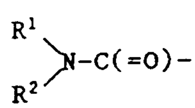

wherein $R^1$ and $R^2$, independently of one another, are each hydrogen, unsubstituted acyclic $C_1$-

$C_7$hydrocarbyl or monocyclic unsubstituted or $C_1$-$C_4$alkyl-, $C_1$-$C_4$alkoxy-, halo- and/or nitro-substituted aryl, aralkyl or aralkenyl having a maximum of 10 carbon atoms, or wherein $R^1$ is hydrogen and $R^2$ is $C_1$-$C_5$alkyl substituted in the 1-position by $C_1$-$C_4$alkoxycarbonyl.

**7.** A process according to claim 3 which comprises reaction with an acylating agent of the formula AcY in which Ac is the radical $O=C=N\text{-}SO_2$- or a radical of the formula lower alkoxy-$(CH_2\text{-}CH_2\text{-}O\text{-})_n$CO-NH-$SO_2$- in which n is from 1 to 19, and Y is bromine or chlorine.

**8.** A process according to claim 3 which comprises reaction with an acylating agent of the formula $R^o$-N=C=O in which $R^o$ is $C_1$-$C_5$alkyl that is unsubstituted or substituted in the 1-position by $C_1$-$C_4$alkoxycarbonyl.

**9.** A process according to claim 3 which comprises reaction with chlorosulfonylisocyanate.

**10.** A process according to claim 3 which comprises reaction with phosgene or bis(1-imidazolyl)-carbonyl.

**11.** A process according to claim 3 which comprises reaction with an acid chloride selected from $C_2$-$C_{20}$-alkanoic acid chloride, benzoyl chloride, $C_{18}$alkenoic acid chloride, chloroformic acid ($C_1$-$C_{12}$alkyl) ester, a chloroformic acid ester of the formula Cl-CO-O-$(CH_2\text{-}CH_2\text{-}O\text{-})_n$-lower alkyl in which n is from 1 to 19, and a chloroformic acid ester of the formula Cl-CO-NH-($C_1$-$C_5$alkylene)-COO-($C_1$-$C_4$alkyl).

**12.** A process according to claim 1 wherein the starting materials are so selected that a compound of formula I

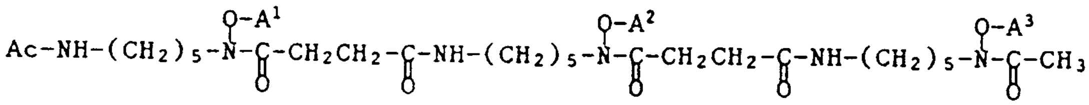

(I)

is manufactured in which Ac is $C_2$-$C_{20}$alkanoyl, benzoyl, $C_{18}$alkenoyl, ($C_1$-$C_{12}$alkoxy)-carbonyl, a radical of the formula -CO-O-$(CH_2\text{-}CH_2\text{-}O\text{-})_n$-lower alkyl in which n is from 1 to 19, chlorocarbonyl, (1-imidazolyl)-carbonyl, N-di-lower alkylcarbamoyl, N-($C_1$-$C_5$alkyl)-carbamoyl substituted in the 1- or 2-position of the alkyl moiety by ($C_1$-$C_4$alkoxy)-carbonyl, N-(chlorosulfonyl)-carbamoyl, N-(lower alkoxysulfonyl)-carbamoyl or a radical of the formula -$SO_2$-NH-CO$(\text{-}O\text{-}CH_2\text{-}CH_2)_n$-lower alkoxy in which n is from 0 to 19, and $A^1$, $A^2$ and $A^3$ are each hydrogen.

**13.** A compound of formula II

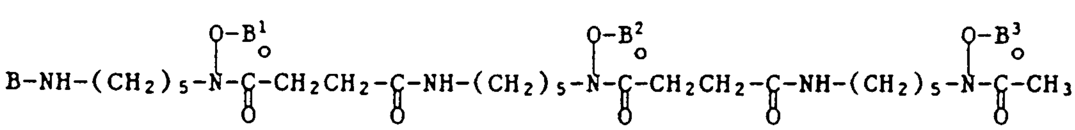

(II)

in which B, $B_o^1$, $B_o^2$ and $B_o^3$ are all an organic silyl group (Sil) of the same meaning of the formula

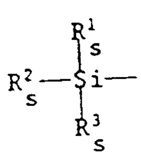

(Sil)

wherein $R_s^1$ and $R_s^2$ are each unsubstituted $C_1$-$C_8$hydrocarbyl and $R_s^3$ is unsubstituted $C_1$-$C_8$hydrocarbyl or chlorine.

**14.** A compound according to claim 13 wherein Sil is dimethylchlorosilyl.

**15.** A compound according to claim 13 wherein Sil is trimethylsilyl.

**16.** The use of a compound according to any one of claims 13-15 as a starting material for the process according to any one of claims 1 to 12.

**Claims for the following Contracting State : ES**

**1.** A process for the introduction of an organic acyl radical selectively at the nitrogen atom of the terminal amino group of desferrioxamine B or of a partially O-acylated derivative thereof, which comprises reacting a compound of formula II

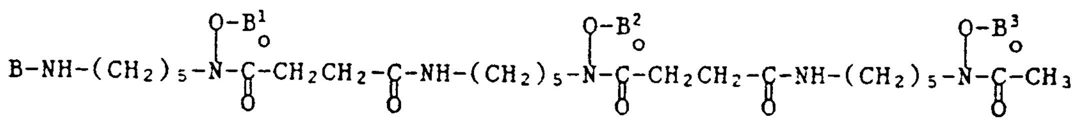

(II)

in which B is an organic silyl group (Sil) of formula

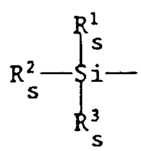

(Sil)

wherein $R_s^1$ and $R_s^2$ , independently of one another, are each unsubstituted $C_{1-8}$hydrocarbyl and $R_s^3$ is unsubstituted $C_{1-8}$hydrocarbyl or chlorine, and at least one of the symbols $B_o^1$ , $B_o^2$ and $B_o^3$ is an organic silyl group Sil and the others, independently of one another, are each an organic silyl group Sil or an organic acyl radical Ac, with an agent that introduces an acyl radical Ac, and removing the O-bonded organic silyl groups Sil present by means of solvolysis.

**2.** A process according to claim 1 which comprises using starting materials of formula II in which B, $B_o^1$ , $B_o^2$ and $B_o^3$ all have the same meaning and are each dimethylchlorosilyl or trimethylsilyl.

**3.** A process according to claim 1 which comprises reaction with an acylating agent of the formula AcY in which Ac is an organic acyl radical that is derived from an optionally functionally modified carboxylic acid, an organic sulfonic acid or an esterified phosphoric acid, and Y is a reactive functionally modified hydroxy group or is an additional bond in the Ac radical that replaces the hydrogen at the atom in the $\alpha$-position to the carbonyl group.

**4.** A process according to claim 3 which comprises reaction with an acylating agent of the formula AcY in which Ac corresponds to the partial formula Z-X-C(=O)- in which X is a single bond, oxy or unsubstituted or substituted imino and Z is hydrocarbyl $R^o$ or, if X is a single bond, also hydrogen, chlorine or 1-imidazolyl.

**5.** A process according to claim 3 which comprises reaction with an acylating agent of the formula AcY in which Ac is characterised by the partial formula $R^o$-O-CO- wherein $R^o$ is $C_1$-$C_{20}$alkyl or a radical of the formula $-(CH_2-CH_2-O-)_n$-lower alkyl in which n is from 1 to 19.

**6.** A process according to claim 3 which comprises reaction with an acylating agent of the formula AcY in which Ac is characterised by the formula

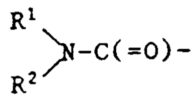

wherein $R^1$ and $R^2$, independently of one another, are each hydrogen, unsubstituted acyclic $C_1$-$C_7$hydrocarbyl or monocyclic unsubstituted or $C_1$-$C_4$alkyl-, $C_1$-$C_4$alkoxy-, halo- and/or nitro-substituted aryl, aralkyl or aralkenyl having a maximum of 10 carbon atoms, or wherein $R^1$ is hydrogen and $R^2$ is $C_1$-$C_5$alkyl substituted in the 1-position by $C_1$-$C_4$alkoxycarbonyl.

**7.** A process according to claim 3 which comprises reaction with an acylating agent of the formula AcY in which Ac is the radical $O=C=N-SO_2$- or a radical of the formula lower alkoxy-$(CH_2-CH_2-O-)_nCO-NH-SO_2$- in which n is from 1 to 19, and Y is bromine or chlorine.

**8.** A process according to claim 3 which comprises reaction with an acylating agent of the formula $R^o$-$N=C=O$ in which $R^o$ is $C_1$-$C_5$alkyl that is unsubstituted or substituted in the 1-position by $C_1$-$C_4$alkoxycarbonyl.

**9.** A process according to claim 3 which comprises reaction with chlorosulfonylisocyanate.

**10.** A process according to claim 3 which comprises reaction with phosgene or bis(1-imidazolyl)-carbonyl.

**11.** A process according to claim 3 which comprises reaction with an acid chloride selected from $C_2$-$C_{20}$-alkanoic acid chloride, benzoyl chloride, $C_{18}$alkenoic acid chloride, chloroformic acid ($C_1$-$C_{12}$alkyl) ester, a chloroformic acid ester of the formula Cl-CO-O-$(CH_2-CH_2-O-)_n$-lower alkyl in which n is from 1 to 19, and a chloroformic acid ester of the formula Cl-CO-NH-($C_1$-$C_5$alkylene)-COO-($C_1$-$C_4$alkyl).

**12.** A process according to claim 1 wherein the starting materials are so selected that a compound of formula I

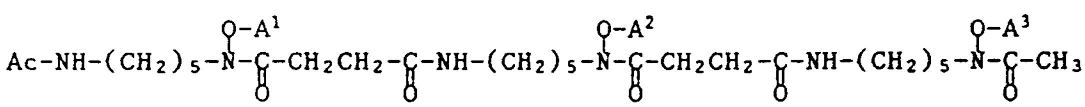

(I)

is manufactured in which Ac is $C_2$-$C_{20}$alkanoyl, benzoyl, $C_{18}$alkenoyl, ($C_1$-$C_{12}$alkoxy)-carbonyl, a radical of the formula -CO-O-$(CH_2-CH_2-O-)_n$-lower alkyl in which n is from 1 to 19, chlorocarbonyl, (1-imidazolyl)-carbonyl, N-di-lower alkylcarbamoyl, N-($C_1$-$C_5$alkyl)-carbamoyl substituted in the 1- or 2-position of the alkyl moiety by ($C_1$-$C_4$alkoxy)-carbonyl, N-(chlorosulfonyl)-carbamoyl, N-(lower alkoxysulfonyl)-carbamoyl or a radical of the formula $-SO_2-NH-CO(-O-CH_2-CH_2)_n$-lower alkoxy in which n is from 0 to 19, and $A^1$, $A^2$ and $A^3$ are each hydrogen.

**13.** A process according to claim 1 wherein a starting material of formula II

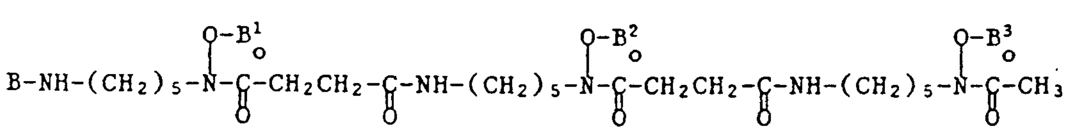

(II)

in which B is an organic silyl group (Sil) of the formula

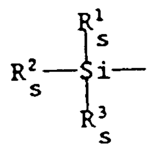

(Sil)

wherein $R_s^1$ and $R_s^2$ , independently of one another, are each unsubstituted $C_{1-8}$hydrocarbyl and $R_s^3$ is unsubstituted $C_{1-8}$hydrocarbyl or chlorine, and at least one of the symbols $B_o^1$ , $B_o^2$ and $B_o^3$ is Sil and the others, independently of one another, are each Sil or an organic acyl radical Ac, is manufactured by reacting a compound of the formula

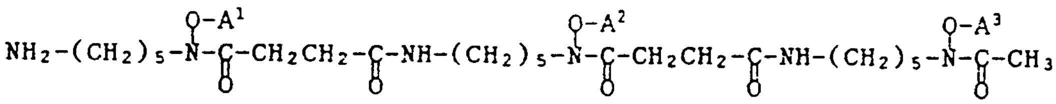

(V)

in which at least one of the symbols $A^1$, $A^2$ and $A^3$ is hydrogen and the others, independently of one another, are each hydrogen or an organic acyl radical Ac, or an acid addition salt thereof, with a silylating reagent of the formula Sil-Hal (VI) in which Sil has the meanings given above and Hal is bromine or chlorine.

**14.** A process according to claim 13 which comprises reacting desferrioxamine B of formula V in which $A^1$, $A^2$ and $A^3$ are all hydrogen, or an acid addition salt thereof, with a tri-lower alkylchlorosilane or di-lower alkyldichlorosilane.

**15.** A process according to claim 13 which comprises reacting desferrioxamine B of formula V in which $A^1$, $A^2$ and $A^3$ are all hydrogen, or an acid addition salt thereof, with trimethylsilyl chloride or dimethyldichlorosilane.

**Revendications**

**Revendications pour les Etats contractants suivants : AT, BE, CH, DE, FR, GB, GR, IT, LI, LU, NL, SE**

**1.** Un procédé pour introduire un radical organique acyle sélectivement sur l'atome d'azote du groupe amino terminal de la desferrioxamine B ou d'un dérivé partiellement O-acylé de cette dernière, caractérisé en ce que l'on fait réagir un composé de formule II

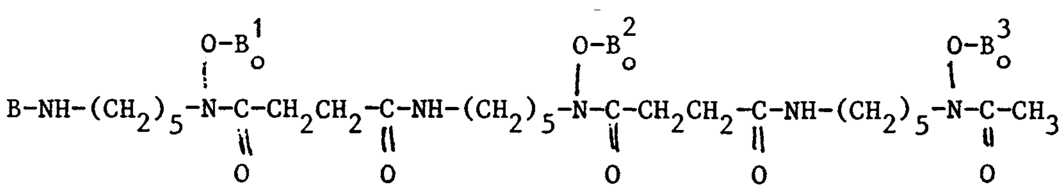

(II)

dans laquelle B représente un groupe organique silyle (Sil) de formule

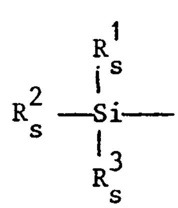

(Sil)

dans laquelle $R_s^1$ et $R_s^2$ représentent chacun, indépendamment l'un de l'autre, un groupe hydrocarboné non substitué en C 1-C 8 et $R_s^3$ représente un groupe hydrocarboné non substitué en C 1-C 8 ou le chlore, et
au moins un des symboles $B_o^1$ , $B_o^2$ et $B_o^3$ représente un groupe organique silyle Sil et les autres, indépendamment l'un de l'autre, un groupe organique silyle Sil ou un radical organique acyle Ac,
avec un agent introduisant un radical acyle Ac, puis on scinde les groupes organiques silyle Sil présents, liés à l'oxygène, par solvolyse.

**2.** Procédé selon la revendication 1, caractérisé en ce que l'on part de composés de formule II dans laquelle B, $B_o^1$ , $B_o^2$ et $B_o^3$ ont tous la même signification et représentent chacun un groupe diméthylchlorosilyle ou triméthylsilyle.

**3.** Procédé selon la revendication 1, caractérisé en ce que l'on fait réagir avec un agent acylant de formule AcY dans laquelle Ac représente un radical organique acyle dérivant d'un acide carboxylique éventuellement fonctionnalisé, d'un acide organique sulfonique ou d'un acide phosphorique estérifié et Y représente un groupe hydroxy fonctionnalisé réactif ou une liaison supplémentaire dans le groupe Ac, remplaçant l'hydrogène sur l'atome en position alpha du groupe carbonyle.

**4.** Procédé selon la revendication 3, caractérisé en ce que l'on fait réagir avec un agent acylant de formule AcY dans laquelle Ac répond à la formule partielle Z-X-C(=O)- dans laquelle X représente une liaison simple, une fonction oxy ou une fonction imino éventuellement substituée et Z représente un groupe hydrocarboné $R^o$ ou bien encore, lorsque X représente une liaison simple, l'hydrogène, le chlore ou un groupe 1-imidazolyle.

**5.** Procédé selon la revendication 3, caractérisé en ce que l'on fait réagir avec un agent acylant de formule AcY dans laquelle Ac est caractérisé par la formule partielle $R^o$-O-CO- dans laquelle $R^o$ représente un groupe alkyle en C 1-C 20 ou un groupe de formule $-(CH_2-CH_2-O-)_n$alkyle inférieur dans laquelle n a une valeur de 1 à 19.

**6.** Procédé selon la revendication 3, caractérisé en ce que l'on fait réagir avec un agent acylant de formule AcY dans laquelle Ac est caractérisé par la formule

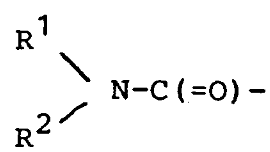

dans laquelle $R^1$ et $R^2$ représentent chacun, indépendamment l'un de l'autre, l'hydrogène, un groupe hydrocarboné acyclique non substitué en C 1-C 7 ou un groupe aryle, aralkyle ou aralcényle monocyclique, éventuellement substitué par des groupes alkyle en C 1-C 4, alcoxy en C 1-C 4, des halogènes et/ou des groupes nitro, à 10 atomes de carbone au maximum, ou bien $R^1$ représente l'hydrogène et $R^2$ un groupe alkyle en C 1-C 5 substitué en position 1 par un groupe (alcoxy en C 1-C 4)-carbonyle.

**7.** Procédé selon la revendication 3, caractérisé en ce que l'on fait réagir avec un agent acylant de formule AcY dans laquelle Ac représente le groupe $O=C=N-SO_2-$ ou un groupe de formule alcoxy inférieur-$(CH_2-CH_2-O-)_nCO-NH-SO_2-$ dans laquelle n a une valeur de 1 à 19, et Y représente le brome ou le chlore.

**8.** Procédé selon la revendication 3, caractérisé en ce que l'on fait réagir avec un agent acylant de formule $R^o-N=C=O$ dans laquelle $R^o$ représente un groupe alkyle en C 1-C 5 non substitué ou substitué en position 1 par un groupe (alcoxy en C 1-C 4)-carbonyle.

**9.** Procédé selon la revendication 3, caractérisé en ce que l'on fait réagir avec le chlorosulfonylisocyanate.

**10.** Procédé selon la revendication 3, caractérisé en ce que l'on fait réagir avec le phosgène ou le bis-(1-imidazolyl)-carbonyle.

**11.** Procédé selon la revendication 3, caractérisé en ce que l'on fait réagir avec un chlorure d'acide choisi parmi les chlorures d'acides alcanoïques en C 2-C 20, le chlorure de benzoyle, les chlorures d'acides alcénoïques en C 18, les chloroformiates d'alkyle en C 1-C 12, les chloroformiates de formule Cl-CO-O-$(CH_2\text{-}CH_2\text{-}O\text{-})_n$alkyle inférieur dans laquelle n a une valeur de 1 à 19, ou les esters chloroformiques de formule Cl-CO-NH-(alkylène en C 1-C 5)-COO-alkyle en C 1-C 4.

**12.** Procédé selon la revendication 1, caractérisé en ce que l'on choisit les composants de départ de manière à préparer un composé de formule I

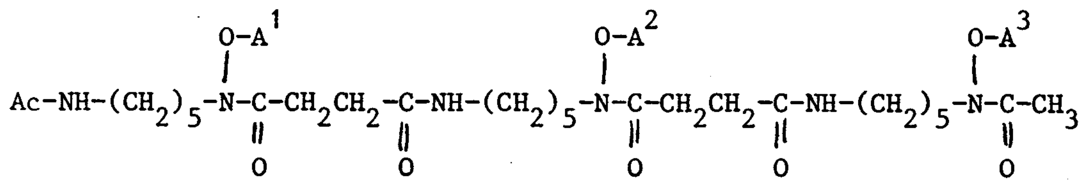

(I)

dans laquelle Ac représente un groupe alcanoyle en C 2-C 20, benzoyle, alcénoyle en C 18, (alcoxy en C 1-C 12)-carbonyle, un groupe de formule -CO-O-$(CH_2\text{-}CH_2\text{-}O\text{-})_n$alkyle inférieur dans laquelle n a une valeur de 1 à 19, un groupe chlorocarbonyle, (1-imidazolyl)-carbonyle, N-di-(alkyle inférieur)-carbamoyle, N-(alkyle en C 1-C 5)-carbamoyle substitué en position 1 ou 2 de la partie alkyle par un groupe (alcoxy en C 1-C 4)-carbonyle, N-(chlorosulfonyl)-carbamoyle, N-(alcoxysulfonyle inférieur)-carbamoyle ou un groupe de formule $-SO_2\text{-}NH\text{-}CO(\text{-}O\text{-}CH_2\text{-}CH_2)_n$alcoxy inférieur dans laquelle n a une valeur de 0 à 19, et $A^1$, $A^2$ et $A^3$ représentent chacun un atome d'hydrogène.

**13.** Un composé de formule II

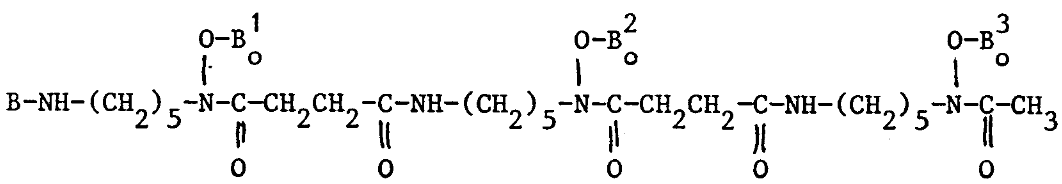

(II)

dans laquelle B, $B^1_o$ , $B^2_o$ et $B^3_o$ représentent tous le même groupe organique silyle Sil de formule

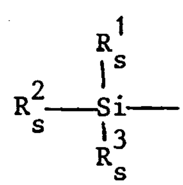

(Sil)

dans laquelle $R^1_s$ et $R^2_s$ représentent chacun un groupe hydrocarboné non substitué en C 1-C 8 et $R^3_s$ représente un groupe hydrocarboné non substitué en C 1-C 8 ou le chlore.

**14.** Un composé selon revendication 13, dans lequel Sil est le groupe diméthylchlorosilyle.

**15.** Un composé selon revendication 13, dans lequel Sil est le groupe triméthylsilyle.

**16.** Utilisation d'un composé selon l'une des revendications 13 à 15 en tant que produit de départ dans le procédé selon l'une des revendications 1 à 12.

**Revendications pour l'Etat contractant suivant : ES**

**1.** Un procédé pour introduire un radical organique acyle sélectivement sur l'atome d'azote du groupe amino terminal de la desferrioxamine B ou d'un dérivé partiellement O-acylé de cette dernière, caractérisé en ce que l'on fait réagir un composé de formule II

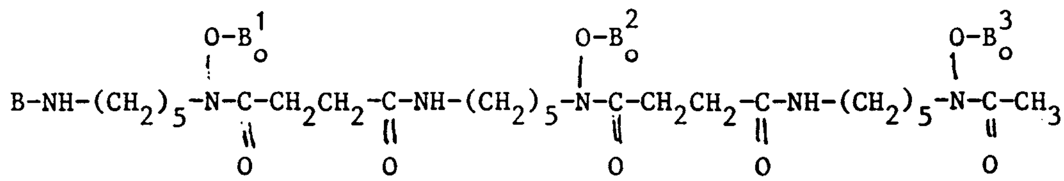

(II)

dans laquelle B représente un groupe organique silyle (Sil) de formule

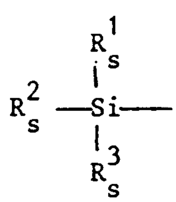

(Sil)

dans laquelle $R_s^1$ et $R_s^2$ représentent chacun, indépendamment l'un de l'autre, un groupe hydrocarboné non substitué en C 1-C 8 et $R_s^3$ représente un groupe hydrocarboné non substitué en C 1-C 8 ou le chlore, et

au moins un des symboles $B_o^1$ , $B_o^2$ et $B_o^3$ représente un groupe organique silyle Sil et les autres, indépendamment l'un de l'autre, un groupe organique silyle Sil ou un radical organique acyle Ac,

avec un agent introduisant un radical acyle Ac, puis on scinde les groupes organiques silyle Sil présents, liés à l'oxygène, par solvolyse.

**2.** Procédé selon la revendication 1, caractérisé en ce que l'on part de composés de formule II dans laquelle B, $B_o^1$ , $B_o^2$ et $B_o^3$ ont tous la même signification et représentent chacun un groupe diméthylchlorosilyle ou triméthylsilyle.

**3.** Procédé selon la revendication 1, caractérisé en ce que l'on fait réagir avec un agent acylant de formule AcY dans laquelle Ac représente un radical organique acyle dérivant d'un acide carboxylique éventuellement fonctionnalisé, d'un acide organique sulfonique ou d'un acide phosphorique estérifié et Y représente un groupe hydroxy fonctionnalisé réactif ou une liaison supplémentaire dans le groupe Ac, remplaçant l'hydrogène sur l'atome en position alpha du groupe carbonyle.

**4.** Procédé selon la revendication 3, caractérisé en ce que l'on fait réagir avec un agent acylant de formule AcY dans laquelle Ac répond à la formule partielle Z-X-C(=O)- dans laquelle X représente une liaison simple, une fonction oxy ou une fonction imino éventuellement substituée et Z représente un groupe hydrocarboné $R^o$ ou bien encore, lorsque X représente une liaison simple, l'hydrogène, le chlore ou un groupe 1-imidazolyle.

**5.** Procédé selon la revendication 3, caractérisé en ce que l'on fait réagir avec un agent acylant de formule AcY dans laquelle Ac est caractérisé par la formule partielle $R^o$-O-CO- dans laquelle $R^o$ représente un groupe alkyle en C 1-C 20 ou un groupe de formule $-(CH_2-CH_2-O-)_n$alkyle inférieur dans laquelle n a une valeur de 1 à 19.

6. Procédé selon la revendication 3, caractérisé en ce que l'on fait réagir avec un agent acylant de formule AcY dans laquelle Ac est caractérisé par la formule

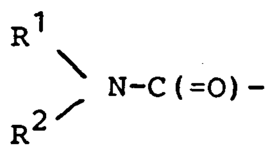

dans laquelle $R^1$ et $R^2$ représentent chacun, indépendamment l'un de l'autre, l'hydrogène, un groupe hydrocarboné acyclique non substitué en C 1-C 7 ou un groupe aryle, aralkyle ou aralcényle monocyclique, éventuellement substitué par des groupes alkyle en C 1-C 4, alcoxy en C 1-C 4, des halogènes et/ou des groupes nitro, à 10 atomes de carbone au maximum, ou bien $R^1$ représente l'hydrogène et $R^2$ un groupe alkyle en C 1-C 5 substitué en position 1 par un groupe (alcoxy en C 1-C 4)-carbonyle.

7. Procédé selon la revendication 3, caractérisé en ce que l'on fait réagir avec un agent acylant de formule AcY dans laquelle Ac représente le groupe $O=C=N-SO_2$- ou un groupe de formule alcoxy inférieur-$(CH_2-CH_2-O-)_n$CO-NH-$SO_2$- dans laquelle n a une valeur de 1 à 19, et Y représente le brome ou le chlore.

8. Procédé selon la revendication 3, caractérisé en ce que l'on fait réagir avec un agent acylant de formule $R^o$-N=C=O dans laquelle $R^o$ représente un groupe alkyle en C 1-C 5 non substitué ou substitué en position 1 par un groupe (alcoxy en C 1-C 4)-carbonyle.

9. Procédé selon la revendication 3, caractérisé en ce que l'on fait réagir avec le chlorosulfonylisocyanate.

10. Procédé selon la revendication 3, caractérisé en ce que l'on fait réagir avec le phosgène ou le bis-(1-imidazolyl)-carbonyle.

11. Procédé selon la revendication 3, caractérisé en ce que l'on fait réagir avec un chlorure d'acide choisi parmi les chlorures d'acides alcanoïques en C 2-C 20, le chlorure de benzoyle, les chlorures d'acides alcénoïques en C 18, les chloroformiates d'alkyle en C 1-C 12, les chloroformiates de formule Cl-CO-O-$(CH_2-CH_2-O-)_n$alkyle inférieur dans laquelle n a une valeur de 1 à 19, ou les esters chloroformiques de formule Cl-CO-NH-(alkylène en C 1-C 5)-COO-alkyle en C 1-C 4.

12. Procédé selon la revendication 1, caractérisé en ce que l'on choisit les composants de départ de manière à préparer un composé de formule I

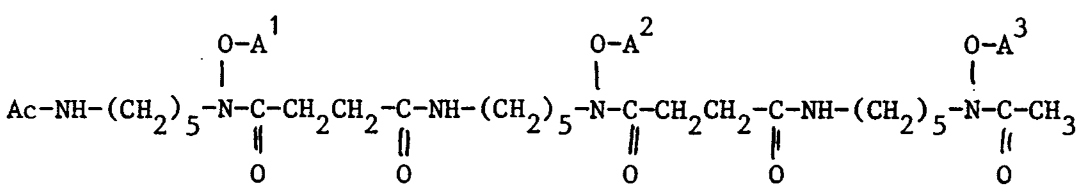

(I)

dans laquelle Ac représente un groupe alcanoyle en C 2-C 20, benzoyle, alcénoyle en C 18, (alcoxy en C 1)C 12)-carbonyle, un groupe de formule -CO-O-$(CH_2-CH_2-O-)_n$alkyle inférieur dans laquelle n a une valeur de 1 à 19, un groupe chlorocarbonyle, (1-imidazolyl)-carbonyle, N-di-(alkyle inférieur)-carbamoyle, N-(alkyle en C 1-C 5)-carbamoyle substitué en position 1 ou 2 de la partie alkyle par un groupe (alcoxy en C 1-C 4)-carbonyle, N-(chlorosulfonyl)-carbamoyle, N-(alcoxysulfonyle inférieur)-carbamoyle ou un groupe de formule -$SO_2$-NH-CO-$(-O-CH_2-CH_2)_n$alcoxy inférieur dans laquelle n a une valeur de 0 à 19, et $A^1$, $A^2$ et $A^3$ représentent chacun un atome d'hydrogène.

**13.** Procédé selon la revendication 1, caractérisé en ce que l'on prépare un composé de départ de formule II

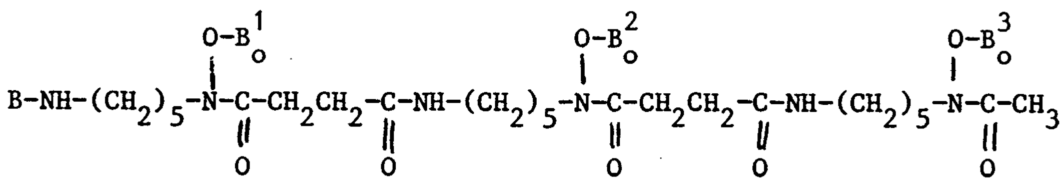

(II)

dans laquelle
B représente un groupe organique silyle (Sil) de formule

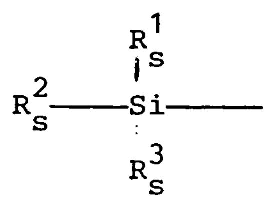

(Sil)

dans laquelle $R_s^1$ et $R_s^2$ représentent chacun, indépendamment l'un de l'autre, un groupe hydrocarboné non substitué en C 1-C 8 et $R_s^3$ représente un groupe hydrocarboné non substitué en C 1-C 8 ou le chlore, et
l'un au moins des symboles $B_o^1$ , $B_o^2$ et $B_o^3$ représente Sil et les autres, indépendamment l'un de l'autre, Sil ou un radical organique acyle Ac, par réaction d'un composé de formule

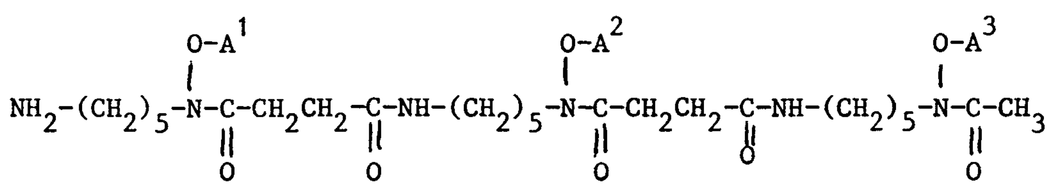

(V)

dans laquelle l'un au moins des symboles $A^1$, $A^2$ et $A^3$ représente l'hydrogène et les autres, indépendamment l'un de l'autre, l'hydrogène ou un radical organique acyle Ac, ou d'un sel d'un tel composé formé par addition avec un acide, avec un réactif silylant de formule Sil-Hal (VI) dans laquelle Sil a les significations indiquées ci-dessus et Hal représente le brome ou le chlore.

**14.** Procédé selon la revendication 13, caractérisé en ce que l'on fait réagir la desferrioxamine B de formule V dans laquelle $A^1$, $A^2$ et $A^3$ représentent tous l'hydrogène, ou un sel de ce composé formé par addition avec un acide, avec un tri-(alkyle inférieur)-chlorosilane ou un di-(alkyle inférieur)-dichlorosilane.

**15.** Procédé selon la revendication 13, caractérisé en ce que l'on fait réagir la desferrioxamine B de formule V dans laquelle $A^1$, $A^2$ et $A^3$ représentent tous l'hydrogène, ou un sel de ce composé formé par addition avec un acide, avec le chlorure de triméthylsilyle ou le diméthyldichlorosilane.